# NEUE EUROPÄISCHE PATENTSCHRIFT

(11) **EP 1 419 240 B2**
(45) Veröffentlichungstag und Bekanntmachung des Hinweises auf die Entscheidung über den Einspruch: **11.07.2018**
(45) Hinweis auf die Patenterteilung: 22.06.2011
(21) Anmeldenummer: 02758474.7
(22) Anmeldetag: 19.08.2002
(51) Int. Cl.: C12N 5/071

(54) **HERSTELLUNG UND VERWENDUNG VON HUMANEN CD124 UND CD116 POSITIVEN TUMORZELLLINIEN ZUR HERSTELLUNG VON ALLOGENEN ODER SEMI-ALLOGENEN IMMUNTHERAPEUTIKA**
PRODUCTION AND USE OF HUMAN CD124 AND CD116 POSITIVE TUMOUR CELL LINES IN THE PRODUCTION OF ALLOGENIC OR SEMI-ALLOGENIC IMMUNOTHERAPY AGENTS
PRODUCTION ET UTILISATION DE LIGNEES DE CELLULES TUMORALES POSITIVES CD124 ET CD116 HUMAINES POUR LA PREPARATION D'AGENTS D'IMMUNOTHERAPIE ALLOGENIQUES OU SEMI-ALLOGENIQUES

(30) Priorität: 17.08.2001 DE 10139428
(43) Veröffentlichungstag der Anmeldung: 19.05.2004
(60) Teilanmeldung: 10185184.8
(73) Patentinhaber: Glycotope GmbH, 13125 Berlin (DE)
(72) Erfinder: GOLETZ, Steffen, 16548 Glienicke/Nordbahn (DE); SCHEPER, Rik, J., NL-1078 LW Amsterdam (NL); MASTERSON, Alan, NL-1058 XV Amsterdam (NL); PINEDO, Herbert, M., NL-1075 HR Amsterdam (NL)
(74) Vertreter: Schiweck, Weinzierl & Koch Patentanwälte Partnerschaft mbB
(86) Internationale Anmeldenummer: PCT/EP2002/009260
(87) Internationale Veröffentlichungsnummer: WO 2003/023023

(56) Entgegenhaltungen:
- US-A- 5 648 219
- US-A- 5 811 297
- GEISSMANN FREDERIC ET AL: "Transforming growth factor beta1, in the presence of granulocyte/macrophage colony-stimulating factor and interleukin 4, induces differentiation of human peripheral blood monocytes into dendritic Langerhans cells" JOURNAL OF EXPERIMENTAL MEDICINE, TOKYO, JP, Bd. 187, Nr. 6, 16. März 1998 (1998-03-16), Seiten 961-966, XP002211123 ISSN: 0022-1007
- JAKSITS S ET AL: "CD34+ cell-derived CD14+ precursor cells develop into Langerhans cells in a TGF-beta 1-dependent manner." JOURNAL OF IMMUNOLOGY (BALTIMORE, MD.: 1950) UNITED STATES 1 NOV 1999, Bd. 163, Nr. 9, 1. November 1999 (1999-11-01), Seiten 4869-4877, XP002217762 ISSN: 0022-1767
- CAUX CHRISTOPHE ET AL: "Respective involvement of TGF-beta and IL-4 in the development of Langerhans cells and non-Langerhans dendritic cells from CD34+ progenitors" JOURNAL OF LEUKOCYTE BIOLOGY, FEDERATION OF AMERICAN SOCIETIES FOR EXPERIMENTAL, US, Bd. 66, Nr. 5, November 1999 (1999-11), Seiten 781-791, XP001040426 ISSN: 0741-5400
- FAIRCHILD P J ET AL: "Directed differentiation of dendritic cells from mouse embryonic stem cells" CURRENT BIOLOGY, CURRENT SCIENCE,, GB, Bd. 10, Nr. 23, 30. November 2000 (2000-11-30), Seiten 1515-1518, XP001104013 ISSN: 0960-9822
- PAGLIA P ET AL: "IMMORTALIZED DENDRITIC CELL LINE FULLY COMPETENT IN ANTIGEN PRESENTATION INITIATES PRIMARY T CELL RESPONSES IN VIVO" JOURNAL OF EXPERIMENTAL MEDICINE, TOKYO, JP, Bd. 178, Nr. 6, 1. Dezember 1993 (1993-12-01), Seiten 1893-1901, XP000572922 ISSN: 0022-1007
- BANCHEREAU J ET AL: "IMMUNOBIOLOGY OF DENDRITIC CELLS" ANNUAL REVIEW OF IMMUNOLOGY, ANNUAL REVIEWS INC, US, Bd. 18, 2000, Seiten 767-811, XP001018258 ISSN: 0732-0582
- OZAWA HIROAKI ET AL: "Granulocyte-macrophage colony-stimulating factor gene transfer to dendritic cells or epidermal cells augments their antigen-presenting function including induction of anti-tumor immunity." JOURNAL OF INVESTIGATIVE DERMATOLOGY, Bd. 113, Nr. 6, Dezember 1999 (1999-12), Seiten 999-1005, XP002227119 ISSN: 0022-202X
- MASTERSON ALLAN J ET AL: "MUTZ-3, a human cell line model for the cytokine-induced differentiation of dendritic cells from CD34+ precursors." BLOOD, Bd. 100, Nr. 2, 15. Juli 2002 (2002-07-15), Seiten 701-703, XP002217763 July 15, 2002 ISSN: 0006-4971
- HU Z-B ET AL: 'Establishment and characterization of two novel cytokine-responsive acute myeloid and monocytic leukemia cell lines, MUTZ-2 and MUTZ-3' LEUKEMIA Bd. 10, 1996, Seiten 1025 - 1040
- AKASHI K. ET AL: 'Effects of Interleukin-4 and Interleukin-6 on the proliferation od CD34+ and CD34- Blasts form acute myelogenous leukemia' BLOOD Bd. 78, 01 Juli 1991, Seiten 197 - 204
- CIGNETTI A. ET AL: 'CD34+ acute myeloid and lymphoid leukemic blasts can be induced to differentiate intob dentritic cells' BLOOD Bd. 94, Nr. 6, 15 September 1999, Seiten 2048 - 2055
- SPAANBROEK R. ET AL: 'IL-4 determines eicosanoid formation in dentritic cells by down-regulation of 15-lipoxygenase 1 expression' PNAS Bd. 98, Nr. 9, 24 Mai 2001, Seiten 5152 - 5157
- QUENTMEIER H. ET AL: 'MUTZ-3, a monocytic model cell line for interleukin-4 and lipopolysaccharide studies' IMMUNOLOGY Bd. 89, 1996, Seiten 606 - 612
- BRUSERUD Ø. ET AL: 'Effects of gamma-irradiation on acute myelogenous leuckemia blasts: In vitro studies of proliferation, constitutive cytokine secretion, and accessory cell function during T cell activation' JOURNAL OF HEMATOTHERAPY & STEM CELL RESEARCH Bd. 8, 1999, Seite 441, XP431
- MIN Y.H. ET AL: 'Differential responses of CD34-positive acute myelogenous leukemic blasts to the costimulating effects of stem cell factor with GM-CSF and/or IL-3' YONSEI MEDICAL JOURNAL Bd. 36, 1995, Seiten 26 - 36
- STEUBE K.G. ET AL: 'Multiple regulation of constitutive and induced interleukin 8 secretion in human myelomonocytic cell lines' CYTOKINE Bd. 12, Nr. 8, August 2000, Seiten 1236 - 1239
- QUENTMEIER H. ET AL: 'Cloning of human thymic stromal lymphopoietin (TSLP) and signaling mechanisms leading to proliferation' LEUKEMIA Bd. 15, 2001, Seiten 1286 - 1292
- CHOUDHURY A. ET AL: 'Dentritic cells derived in vitro from acute myelogenous leukemia cells stimulate autologous, antileukemic T-cell responses' BLOOD Bd. 93, Nr. 3, 01 Februar 1999, Seiten 780 - 786
- EIBL B. ET AL: 'Dentritic cells generated from blood precursors of chronic myelogenous leukemia patients carry the Philadelphia translocation and can induce a CML-specific primary cytotoxic T-cell response' GENES, CHROMOSOMES AND CANCER Bd. 20, 1997, Seiten 215 - 223 & HARRISON B.D. ET AL: 'Stimulation of autologous proliferative and cytotoxic T-cell responses by "leukemic dentritic cells" derived from blasts cells in acute myeloid leukemia' BLOOD Bd. 97, Nr. 9, 01 Mai 2001, Seiten 2764 - 2771
- DREXLER H.G. ET AL: 'Cytokine response profiles of human myeloid factor-dependent leukemia cell lines' LEUKEMIA Bd. 11, 1997, Seiten 701 - 708
- STEUBE K.G. ET AL: 'Constitutive protein expression of monocyte chemotactic protein-1 (MCP-1) by myelomonocytic cell line and regulation of the secretion by anti-and proinflammatory stimuli' LEUKEMIA RESEARCH Bd. 23, 1999,
- BERNHARD H. ET AL: 'Generation of immunostimulatory dentritic cells form human CD34+ hematopoietic progenitor cells of the bone marrow and pheripheral blood1' CANCER RESEARCH Bd. 55, 01 März 1995, Seiten 1099 - 1104
- KOSKI G.K. ET AL: 'Calcium ionophore-treated myeloid cells acquire many dentritic cell characteristics independent of prior differentiation state, transformation status, or sensitivity to biologic agents' BLOOD Bd. 94, Nr. 4, 15 August 1999, Seiten 1359 - 1371
- OEHLER L. ET AL: 'Culture requirements for induction of dentritic cell differentiation in acute myeloid leukemia' ANN. HEMATOL. Bd. 79, 2000, Seiten 355 - 362
- MACLEOD R.A.F. ET AL: 'Cohabiting t(12;22) and inv(3) primary rearrangements in ana acute myelomonocytic leukemia (FAB M4) cell line' GENES, CHROMOSOMES & CANCER Bd. 16, 1996, Seiten 144 - 148
- Excerpt from Sigma Aldrich catalogue online: www.sigmaaldrich.co.
- NICOLÒ C. ET AL: 'UVB-induced apoptosis of human dentritic cells: contribution by caspase-dependent and caspase-independent pathways' BLOOD Bd. 97, Nr. 6, 15 März 2001, Seiten 1803 - 1808
- STEINMAN R.M.: 'The dentritic cell system and its role in immunogenicity1' ANN. REV. IMMUNOLOGY Bd. 9, 1991, Seiten 271 - 296
- THURNER B. ET AL: 'Generation of large numbers of fully mature and stable dentritic cells from leukapheresis products for clinical application' JOURNAL OF IMMUNOLOGICAL METHODS Bd. 223, 1999, Seiten 1 - 15
- KOEFFLER H.P.: 'Phorbol ester effect on differentiation of human myeloid leukemia cell lines blocked at different stages of maturation1' CANCER RESEARCH Bd. 41, März 1981, Seiten 919 - 926
- ROLLINS B.J.: 'Chemokines' BLOOD Bd. 90, Nr. 3, 01 August 1997, Seiten 909 - 928
- SANTEGOETS S.J.A.M. ET AL: 'Human dentritic cell line models for DC differentiation and clinical DC vaccination studies' JOURNAL OF LEUKOCYTE BIOLOGY Bd. 84, Nr. 6, Dezember 2008, Seiten 1364 - 1373
- Reseach report by Prof. Dr. A. Kruisbeek
- FONG L. ET AL: 'Dentritic cells in cancer immunotherapy' ANNU. REV. IMMUNOLOGY Bd. 18, 2000, Seiten 245 - 273
- ROSENZWAIG M. ET AL: 'Human dentritic cell differentiation pathway from CD34+ hematopoietic precursor cells' BLOOD Bd. 87, Nr. 2, 15 Januar 1996, Seiten 535 - 544
- BERNHARD H. ET AL: 'Generation of immunostimulatory dentric cells of human CD34+ hematopoietic progenitor cells of the bone marrow and peripheral blood' CANCER RESEARCH Bd. 55, 01 März 1995, Seiten 1099 - 1104
- MACKENSEN A. ET AL: 'Delineation of the dentritic cell lineage by generating large numbers of birbeck granule-positive Langerhans cells from human peripheral blood progenitor cells in Vitro' 86 Bd. 7, 01 Oktober 1995, Seiten 2699 - 2707
- BAUMEISTER H. AT AL: 'Voll funktionsfähige humane dentritische Zellinie' LABORWELT Nr. 1, 2005, Seiten 10 - 14
- CAO Y. ET AL: 'The fucosylated histo-blood group antigens H type 2 (blood group 0,CD173) and Lewis Y (CD174) are expressed on CD34+ hematopoietic progenitors but absent on mature lymphocytes' GLYCOBIOLOGY Bd. 11, Nr. 8, 2008, Seiten 677 - 683
- VIEIRA P.L. ET AL: 'Development of Th1-inducing capacity in myeloid dentritic cells requires environmental instruction' J. IMMUNOLOGY Bd. 164, 2000, Seiten 4507 - 4512
- DAVIS T.A.: 'Phorbol esters induce differentiation of human CD34+ hemopoietic progenitors to dentritic cells: Evidence for protein Kinase C-mediated signaling' J. IMMUNOLOGY Bd. 160, 1998, Seiten 3689 - 3697
- Print-out from dejure.org re:EGBGB
- Excerpt from the Commercial Register HRB49306
- Excerpt from the Commerciaq Register HRB83234
- BEYER M. ET AL: 'High-resolution transcriptome of humanmacrophages' PLOS ONE Bd. 7, Nr. 9, September 2012, Seiten 1 - 16

## Beschreibung

Die Erfindung beschreibt die Herstellung von effektiven dendritischen Zellen (DC) oder Zelllinien aus der CD124+ und CD116+ MUTZ-3 Zelllinie mit Hilfe der stimulatorischen Moleküle GM-CSF, TNF-alpha, LPS, PGE2, CD40 Ligand, polylC, PMA, TGF-betal, IL-7, und/oder IL-4 und dabei erhältliche effektive dendritische menschliche MUTZ-3 Zellen oder eine MUTZ-3 Zelllinie.

Dendritische Zellen (DC) spielen eine wichtige Rolle als Antigenpräsentierende Zellen (APC). Sie übermitteln costimulatorische Signale, die für die T-Zell-Aktivierung notwendig sind, und induzieren primäre Immunantworten durch die Präsentation von Antigenen für CD4⁺- und CD8⁺ - T-Zellen (Banchereau et al. 1998, Nature 392 (6673): 245-252). DC entwickeln sich aus hämatopoetischen Vorläuferzellen im Knochenmark und durchlaufen sequentiell verschiedene Differenzierungsstufen (intermediäre Vorläuferzellen im Blut und unreife DC in den peripheren Geweben und Organen) (Banchereau et al. 2000, Ann Rev Immunol 18: 767-811). Einmal im Gewebe angelangt erfüllen unreife DC (iDC) eine wichtige Sensorfunktion, die durch eine hohe aktive Aufnahme von Antigenen aus dem umgebenden Milieu gekennzeichnet ist. Nach ihrer Stimulation durch externe Signale ("Danger Signals") wie z.B. bakterielle oder virale Infektionen oder Entzündungen migrieren die DC in die peripheren lymphatischen Organe, wo sie zu reifen DC ausdifferenzieren und mittels Antigen-Präsentation T-Zellen aktivieren.
Geissmann et al. (1998), J. Exp. Med. 187: 961-966; beschreibt, dass TGF-beta 1 und **IL**-4 die Differenzierung von menschlichen peripheren Blutmonocyten zu dendritischen Langerhanszellen induzieren.
Ebenso beschreibt Jaksits et al. (1999), J. Immunology 163:4869-4877 die Differenzierung von CD34+ Zellen abgeleiteter CD14+ Vorläuferzellen zu Langerhanszellen in Anwesenheit von GM-CSF/TNF-alpha und TGF-beta 1. Auch Caux et al. (1999), J. Leukoc. Biol. 66:781-791 berichtet, dass sich CD34+ Vorläuferzellen in Anwesenheit von TGF-beta und IL-4 zu Langerhanszellen und Nicht-Langerhans dendritischen Zellen entwickeln können.
US 5,811,297 beschreibt die Kultivierung immortalisierter CD34+ hematopoetischer Stammzellen in Anwesenheit von GM-CSF, TNF-alpha und IL-4, wobei diese Zellen zu dendritischen Zellen reifen.
US 5,648,219 beschreibt die dendritische JAWS II-Maus-Zelllinie, die z.B. mit TNF-alpha, GM-CSF oder IL-4 induziert werden kann.
Fairchild et al. (2000), Current Biology 10:1515-1518 beschreibt, dass dendritische Zellen aus embryonalen Stammzellen der Maus, z.B. in Anwesenheit von IL-3 oder GM-CSF erhalten werden können.
Paglia et al. (1993), J. Exp. Med. 178:1893-1901 berichtet, dass eine immortalisierte dendritische Zelllinie, die Antigene präsentiert, in vivo eine primäre T-Zellaktivität hervorrufen kann.
Hu et al. (1996), Leukemia 10: 1025-1040 beschreibt die Zelllinien MUTZ-2 und MUTZ-3 und merkt an, dass diese Zelllinien für Differenzierungsstudien verwendet werden können. Diese Anmerkung ist allgemein gehalten und stellt keinen Bezug zu dendritischen Zellen her.

Nach bisherigen Methoden zur Herstellung von DC in vitro erhält man zwei Hauptpopulationen von DC- Vorläuferzellen: CD1a⁺/CD14⁻ -Zellen, die sich zu Langerhans-Zellen (LC) entwickeln, und CD1a⁻/CD14⁺ -Zellen, die zu interstitiellen DC ausdifferenzieren. Monozyten können nach der Kultivierung mit GM-CSF und IL-4 einen Phänotyp entwickeln, der dem unreifer DC (iDC) ähnelt. Eine weitere Differenzierung und Reifung wird durch verschiedene Stimuli, wie bakterielle Lipopolysaccharide (LPS), TNFalpha, PGE2, CD40-Ligand oder PolylC erreicht. Die bisher verfügbaren, definierten Kultursysteme wurden für Untersuchungen zur Biologie der DC eingesetzt. Ihr Einsatz für Experimente im großen Maßstab ist aber limitiert durch die Abhängigkeit von verfügbarem Spendermaterial und dessen Variabilität. Im Maussystem haben sich Zytokin (GM-CSF)-abhängige dendritische Zellinien als sehr wertvoll für das Studium der DC-Differenzierung und -Entwicklung in in vitro und in vivo Krankheitsmodellen erwiesen. Solche Zelllinien wurden durch Immortalisierung von murinem Lymph- oder Hautgewebe gewonnen. Sie repräsentieren einen unreifen DC-Phänotyp, der invariabel ist und erlauben daher nicht die Untersuchung verschiedener Faktoren, die an der Differenzierung von DC beteiligt sind. Außerdem lassen sich aufgrund der Heterogenität von DC im murinen und humanen System wenn überhaupt, dann nur sehr begrenzt Aussagen zur humanen DC-Biologie treffen.

Es konnte beobachtet werden, daß Tumoren lymphoiden oder myeloiden Ursprungs Gemeinsamkeiten mit APC in der Ontogenese aufweisen. Studien mit PBMC von Patienten mit chronisch myeloischer Leukämie (CML) und akkuter myeloischer Leukämie (AML) haben gezeigt, daß in Subpopulationen von CML und AML-Blasten durch Zytokine eine in Teilen DC-ähnliche Differenzierung erzielt werden kann, die zum Teil eine verstärkte APC-Funktion aufweisen. Daraufhin wurde versucht etablierte leukämische Zelllinien als in vitro Modellsysteme für untersuchungen zur DC Biologie zu verwenden. Dies gelang allerdings nicht, da alle untersuchten Zelllinien nur bestimmte Stadien der DC Entwicklung erreichen konnten, über die hinaus sie nicht weiter differenzieren konnten, und damit die DC Biologie nicht wie gewünscht wiederspiegeln. Dies ist darauf zurück zu führen, dass die Fähigkeit solcher maligner Zellen, auf Zytokinstimuli zu antworten, von der Expression spezifischer und funktioneller Rezeptoren abhängig ist. Viele Leukämie-Zellinien reagieren jedoch nicht auf eine Zytokin-Behandlung. Andere bisher untersuchte Leukämie-Zelllinien reagieren nur auf die Behandlung mit einzelnen Zytokinen und können nicht durch eine sequentielle DC Differenzierung zu effetiven DC gebracht werden. Pharmakologische Agenzien, die intrazelluläres Kalzium mobilisieren und damit defekte Rezeptor-Signalwege umgehen, können zwar verwendet werden, um einen DC-ähnlichen Phänotyp in myeloiden Zellen zu induzieren, und die Aktivierung von Proteinkinase C durch PMA induziert einen DC-Phänotyp in der humanen Myeloblasten-Zellinie KG-1, allerdings führt die Manipulation intrazellulärer Signalwege mit solchen Agenzien zu APC, die nicht die volle DC-Funktion ausfüllen können. Im Fall der Zytokinstimulierten KG-1 wurde so z.B. keine Differenzierung ohne sofortige Reifung beobachtet werden.

Damit sind die bisher untersuchten Zelllinien für Untersuchungen der DC Biologie nur sehr begrenzt geeignet. Für Immuntherapeutikaanwendungen und Testsyteme für die Testung von Substanzen, die auf das Immunsystem Einfluß nehmen sind diese nicht geeignet. Der Stand der Technik war daher, dass leukämische Zelllinien oder andere Tumorzelllinien nicht in der Lage sein sollten durch entsprechende Stimulation zu unreifen DC, die entsprechend der Stimulation entweder interstitielle DC oder Langerhans DC ähnlich sind, und darauffolgend zu potenten reifen DC, gezielt entweder DC Typ1 oder DC Typ2, zu differenzieren.

Zur Zeit werden DC in unterschiedlichen Verfahren und Ansätzen für die Behandlung verschiedener Erkrankungen eingesetzt, darunter beispielsweise Tumorerkrankungen, Infektionserkrankungen und Autoimmunerkrankungen. Die. Ergebnisse hierfür sind erfolgreich und vielversprechend. Allerdings müssen zur Zeit für alle Behandlungen DC eingesetzt werden, die aus Primärzellen gewonnen werden, da es bisher trotz großer Bemühungen nicht gelungen ist Zelllinien zu generieren und zu identifizieren, aus denen DC erzeugt werden können, die eine effektive Immunantwort stimulieren. Die Nachteile von DC aus Primärzellen sind beispielsweise, dass sie, beziehungsweise ihre Vorläuferzellen, nur in sehr geringen Mengen von Patienten oder Spendern gewonnen werden können, die den Einsatz der Zellen stark limitieren, ihre Gewinnung ist von zeitlich und arbeitstechnisch großem Aufwand, die Menge der gewonnenen DC ist so gering, dass bei weitem nicht die Mengen proportional im Menschen eingesetzt werden können, die in Maus-Modellen die besten Behandlungserfolge erzielen können. Die DC müssen dabei aus Vorläufer-Zellen, wie beispielsweise CD34 positiven Stammzellen oder Monozyten, gewonnen werden, die in vitro durch eine geeignete Stimulierung mit stimulatorischen Molekülen zu DC reifen, wobei die Vorläufer-Zellen sowohl im Blut wie auch im Gewebe äußerst selten vorkommen. Es wird geschätzt, dass sie etwa 1% der PBMC darstellen. Außerdem ist ihre Kultur schwierig und wird durch die aus den PMBC gewonnene Menge an Monozyten stark limitierend beeinflusst und häufig durch Verunreinigungen an Progenitor Zellen gestört. Die resultierende große Varianz in der Reinigungs-, Stimulierungs und Effektivitätseffizienz von autologen DC-Vorläuferzellen erschweren eine Standardisierung von Verfahren für Immuntherapeutische Behandlungen sehr. Zu der Varianz innerhalb eines Patienten kommt noch die Varianz zwischen den einzelnen Individuen hinzu.

Für die Entwicklung von Immuntherapeutika auf der Basis von effektiven DC ist es vorteilhaft genau charakterisierte Zellinien zu generieren, die entweder effektive DC darstellen oder durch geeignete Stimulierung mit geeigneten Signalmolekülen in vitro sich zu solchen wandeln lassen, die dann alleine oder in Kombination mit anderen Stoffen effektive Immuntherapeutika ergeben.

Die erfindungsgemäße Aufgabe ist es daher, ein Verfahren zur Herstellung von Zelllinien oder Zellen bereitzustellen, aus denen sich effektive dendritische Zellen(DC) generieren lassen, die insbesondere als Immuntherapeutika oder als Teil von Immuntherapeutika zur Behandlung von Immunerkrankungen eingesetzt werden können.

Die Erfindung löst dieses technische Problem durch die Bereitstellung eines Verfahrens zur Herstellung von effektiven dendritischen Zellen oder Zelllinien, wobei die CD124 und CD 116 positive MUTZ-3 Zelllinie mit stimulatorischen Molekülen zeitgleich oder sequentiell zeitversetzt in Kontakt gebracht und die effektiven dendritischen Zellen oder Zelllinien erhalten werden, wobei die stimulatorischen Moleküle GM-CSF, TNF-alpha, LPS, PGE2, CD40 Ligand, polylC, PMA, TGF-beta1, IL-7und/oder IL-4 sind.

Im Zusammenhang mit der Erfindung sollen folgende Begriffe wie folgt verwendet werden:
Unter **Zelllinien** aus denen effektive dendritische Zellen (effektive DC) gewonnen werden fallen alle Tumorzelllinien, bevorzugt leukämische Zelllinien, wie beispielsweise myeloide, lymphoide und plasmacytoide, und solche Zelllinien, die nicht leukämischen Ursprungs sind, aber CD124 und CD116, bevorzugt auch CD34, tragen, einschließlich solcher Zelllinien die nicht Tumorzelllinien im strengen Sinne sind. Dabei kann es sich auch um Zelllinien handeln, denen CD124 und/oder CD116 fehlt, die aber durch ein Einbringen von Genen funktionelle rekombinante CD124 und CD116 exprimieren die es ermöglichen effektive DC herzustellen. Vorzugsweise sind Zelllinien, aus denen effektive DC gewonnen werden auch positiv für CD34, wobei CD34 auch durch Gene eingebracht werden kann. Solche Zelllinien, aus denen effektive DC hergestellt werden können aus Tumorzellen oder Primärzellen gewonnen werden. Dies geschieht durch an sich herkömmliche Verfahren, wie beispielsweise Transformation, Immortalisierung, Zellfusion mit Tumorzellen und/oder durch Kultivierung in vitro und/oder in vivo mit oder ohne Klonierung der Zellen möglichst homogenen Zelllinien. Bevorzugt werden dabei Verfahren bei denen die CD124 und CD116 positiven Zellen durch Magnetkugel Techniken oder Cell-Sorting im FACS nach an sich bekannten Methoden angereichert und kloniert werden. Als Spender für Tumorzellen, die erfindungsgemäß durch stimulatorische Moleküle zu Zelllinien gebracht werden, aus denen effektive DC gewonnen werden, werden Patienten mit chronischer myeloischer Leukämie oder akuter myeloischer Leukämie bevorzugt, die Erfindung beschränkt sich jedoch nicht darauf. Primärzellen aus denen geeignete Zelllinien gewonnen werden sind bevorzugt myeloiden, lymphoiden, plasmacytoiden oder monocytären Ursprungs. Um effektive DC aus Zelllinien zu gewinnen und /oder die Effektivität der gewonnenen DC zu steigern, können in die Zelllinien, Tumorzellen oder Primärzellen ein oder mehrere Gene nach an sich bekannten Methoden eingebracht, die beispielsweise Rezeptoren oder Inhibitoren für stimulatorische Moleküle codieren und/oder exprimieren. Als Gene können auch ein oder mehrere Immuntherapeutika eingebracht werden. Das Einbringen der Immuntherapeutikagene in diesem Stadium der Zelllinie hat den Vorteil, dass sie als Zelllinie charakterisiert werden können und für eine weitere Verwendung als Immuntherapeutika nicht nach der Reifung zu dendritischen Zellen eingebracht werden müssen.Eine weitere Möglichkeit der Einbringung von Genen ist die Fusion der Zelllinien mit anderen Zellen oder Zelllinien nach an sich bekannten Methoden.

Die Zelllinie, die im erfindungsgemäßen Verfahren zur Herstellung von effektiven denddritischen Zellen oder Zelllinien verwendet wird, ist die CD124 und CD116 positive MUTZ-3 Zelllinie.

Unter **effektiven DC** versteht man erfindungsgemäß solche Zellen oder Zelllininen, die durch Stimulation von Zelllinien mit stimulatorischen Molekülen zu Zellen differenziern, die wie dendritische Zellen agieren und humorale und/oder zelluläre Teile des Immunsystems aktivieren, inhibieren oder modulieren. Die effektiven DC werden als Immuntherapeutika eingesetzt. Hierfür werden die effektiven DC, ihre Vorläuferzellen in den geeigneten Differenzierungsstadien oder die Zellen der Zelllinien mit mindestens einem Antigen beladen. Die Beladung erfolgt nach an sich bekannten Methoden beispielsweise durch Beladung mit synthetischen oder aus biologischem Material gereinigten oder partiell gereinigten Tumorantigenen oder Infektionsantigenen, mit Zelllysaten von Tumorzellen, Tumorzelllinien, infizierten Zellen oder Zelllinien, durch Fusion mit anderen Zellen oder Zelllinien, durch Einbringung von mindestens einem Immuntherapiegen, durch Infektion mit infektiösen Partikeln oder jeweils teilen daraus. Gegebenenfalls werden die beladenen Zellen oder Zelllinien weiter durch stimulatorische Moleküle differenziert. Die effektiven DC prozessieren in der Regel die Antigene und präsentieren sie über bestimmte Moleküle den entsprechenden Immunzellen des Immunsystems, bespielsweise über MHCI oder MHCII Moleküle, und aktivieren dadurch entsprechend die humorale und/ oder zelluläre Immunanwort, die die Krankheit bekämpft oder ein immunologisches Memory aufbaut, das prophylaktisch Krankheiten verhindert. Die effektiven DC werden hierfür in mindestens einem geeigneten Aktivitäts- und/oder Effektorstadium im Patienten als Immuntherapeutikum eingesetzt.

Effektive dendritische Zellen der Erfindung sind aus der MUTZ-3 Zelllinie erhältlich.

Unter **stimulatorischen Molekülen** versteht solche chemischen und biologischen Moleküle, die eine Differenzierung von Zellen beeinflussen wie beispielsweise, Cytokine (IL-4, TNFalpha), Wachstumsfaktoren (z.B. GM-CSF), surrogat Moleküle für Cytokine oder Wachstumsfaktoren,, die einen vergleichbaren biologischen Effekt induzieren wie die stimulatorischen Moleküle selbst, beispielsweise Antikörper, andere biologische Moleküle (z.B. LPS, polylC) und chemische Agenzien. Die Moleküle können zeitgleich gemeinsein oder sequentiell zeitversetzt angewandt werden um das entsprechende gewünschte Differenzierungsstadium der Zellen und damit unterschiedliche Aktivitäts- und Effektorstadien zu erreichen, beispielsweise DC Typ1 oder DC Typ 2 Phänotypische Zellen, die je nach Eignung für die verschiedenen Verwendungen eingesetzt werden können. Beispielsweise können so durch verschiedene stimulatorische Moleküle aus der gleichen Tumorzellausgangslinie unterschiedlich effektive DC hergestellt werden, die beispielsweise inhibitorisch oder stimulatorische auf verschiedene Komponenten des Immunsystems wirken und damit beispielsweise entweder in der Immuntherapie von Infektionserkrankungen und Tumorerkrankungen oder Autoimmunerkrankungen eingesetzt werden. Unter stimulatorische Moleküle fallen erfindungsgemäß außerdem alle Danger Signale einschließlich solcher, die streng genommen keine Moleküle sind, wie beispielsweise mechanischer Streß. Die stimulatorischen Moleküle, die im erfindungsgemäßen Verfahren verwendet werden, sind GM-CSF, TNF-alpha, LPS, PGE2, CD40 Ligand; polylC, PMA, TGF-beta1, IL-7, und/oder IL-4.

Unter **Immuntherapeutika** versteht man im Sinne der Erfindung:
- solche Therapeutika, die gegen Erkrankungen prophylaktisch oder kurativ eingesetzt werden, bei denen effektive dendritische Zellen für die Behandlung eingesetzt werden können, wobei die geeigneten effektiven dendritischen Zellen unterschiedlicher Entwicklungs- und Aktivierungsstadien sein können. Die Behandlungserfolg kann dabei vollständig oder partiell sein. Dabei kann es sich beispielsweise um Vakzinen handeln.

**Semi-allogene DC** für Immuntherapeutika sind erfindungsgemäß solche effektiven DC, die in einem oder mehreren der HLA Moleküle mit dem Empfänger der Immuntherapeutika übereinstimmen und die Zellen nicht von der gleichen Person stammen. Damit sind auch solche DC enthalten die eine komplette übereinstimmung in den HLA Molekülen aufweisen und nicht von der gleichen Person stammen.

**Allogene DC** für Immuntherapeutika sind erfindungsgemäß solche effektiven DC, die in keinem der HLA Moleküle mit dem Empfänger der Immuntherapeutika übereinstimmen.

Unter **CD124 positiven Zelllinien oder Zellen** versteht man erfindungsgemäß solche Zellen, die gegenüber einer Behandlung mit IL-4 sensitiv sind.

Unter **CD116 positiven Zelllinien oder Zellen** versteht man erfindungsgemäß solche Zellen, die gegenüber einer Behandlung mit GM-CSF sensitiv sind.

Unter **Inmuneckrankungen** versteht man erfindungsgemäß alle Erkrankungen, bei denen dendritische Zellen zur Behandlung eingesetzt werden können, beispielsweise:
- Infektionskrankheiten
- Tumorerkrankungen
- Autoimmunerkrankungen.

Unter dem **Einbringen von Genen** versteht man erfindungsgemäß, eine Transfektion oder virale Infektion oder Transformation von Zellen oder Zelllinien wodurch genetisches Material in die Zelle oder Zelllinien nach an sich bekannten Methoden eingebracht wird. Das genetische Material kann dabei DNA oder RNA sein. Das genetische Material codiert für die Expression von mindestens einem Protein oder Peptid oder/und die RNA selbst kann dabei inhibitorisch oder stimulatorisch, beispielsweise als Antisense-RNA, wirken. Die exprimierten Proteine können weiter prozessiert und modifiziert werden, beispielsweise durch Glykosylierung. Gene können auch dadurch eingebracht werden, dass die Zellen oder Zelllinien mit anderen Zellen oder Zellinien fusioniert werden.

**Immuntherapeutikagene** sind erfindungsgemäß Gene,die für Proteine und/ oder Peptide kodieren, die für die Verwendung der effektiven dendritischen Zellen als Immuntherapeutika eine Rolle spielen, beispielsweise Tumorantigene, virale Antigene oder Antigene von Parasiten, Bakterien oder anderen Mikroorganismen. Zellen oder Zelllinien in die Immuntherapeutikagene eingebracht wurden exprimieren die Proteine oder Peptide dieser Gene. Diese werden dann von den dendritischen Zellen dem Immunsystem präsentiert, wodurch die effektiven dendritischen Zellen entsprechende Immunantworten abhängig von dem Aktivitäts - und Effektorstadien der effektiven dendritischen Zellen aktivieren, inhibieren oder modulieren. Für die Präsentation der Genprodukte werden die exprimierten Proteine oder Peptide prozessiert oder direkt verwendet, außerdem können die exprimierten Proteine oder Peptide modifiziert werden, beispielsweise durch Glykosylierung.

**Surrogat-Moleküle** sind solche Moleküle, die die stimulatorischen Moleküle wirkungsmäßig ersetzen können, beispielsweise können anstatt von Cytokinen Antikörper oder Mimikry-Peptide verwendet werden, die die Zellen ebenfalls wie stimulatorische Moleküle beeinflussen.

Um Zellen erfindungsgemäß in die **Apoptose oder Nekrose** zu bringen, werden je nach Bedarf unterschiedliche Verfahren angewendet, beispielsweise Bestrahlung, Hitzeschock, mechanischer Streß, oxidativer Streß, Ultraschall, Induktion von Suizidgenen, Induktion durch chemische und biologische Moleküle, Glyzerin, Zink, Butilinsäure, Natriumbutyrat, Leptomycin B mit STI571 und/oder Fas-Ligandgebracht werden. Dabei können die Zellen auch Mischpopulationen bilden, von denen Teile in die Apoptose oder Nekrose gehen. Dieses Verfahren kann angewendet werden um sicherzustellen, daß effektive dendritische Zellen im Organismus nicht lebensfähig sind.

**Tumorantigene** sind erfindungsgemäß Peptide, Proteine, Lipide, Lipopeptide, Lipoproteine, Kohlenhydrate, Glykolipide, Glykopeptide, Glykoproteine, phosphorylierte Proteine, phosphorylierte Peptide, anderweitig posttranslational modifizierte Proteine oder Peptide, die in den Zellen des Tumors vergleichend zum Normalgewebe, überexprimiert werden, unterexprimiert werden, de novo exprimiert werden, mutiert sind, differentiell posttranslational modifiziert sind, differenziell prozessiert werden, differenziell lokalisiert werden, differenziell gefaltet werden, oder andersweitig verändert sind.

**Infektionsantigene** sind erfindungsgemäß Peptide, Proteine, Lipide, Lipopeptide, Lipoproteine, Kohlenhydrate, Glykolipide, Glykopeptide, Glykoproteine, phosphorylierte Proteine, phosphorylierte Peptide, anderweitig posttranslational modifizierte Proteine oder Peptide, die vom infektiösen Partikel stammen oder davon abgeleitet sind.

**Infektiöse Partikel** sind erfindungsgemäß infektiöse Einheiten, die Krankheiten verursachen oder davon abgeleiteten Teilen. Hierzu gehören beispielsweise Viren, Bakterien, Parasiten und Prionen. Die infektiösen Partikel, die erfindungsgemäß zur Herstellung von effektiven dendritischen Zellen und ihrer Verwendung dienen sind in vivo im Patienten nicht vermehrungsfähig.

Außerdem ist hierin die Verwendung und Herstellung von CD124-+ und CD116+ Tumorzelllinien beschrieben, die bevorzugt auch CD34+ sind, als Modell- und Testsysteme für Testung der DC-Biologie und für die Testung von Substanzen, die auf das Immunsystem und dessen Konditionierung Einfluss nehmen.

Unter einem **Modell- und Testsystem für die Testung der DC-Biologie** versteht man Testsysteme, die als Komponente eine CD124+ und CD116+ Tumorzellinie haben, die bevorzugterweise auch CD34+ ist, und mit deren Hilfe Vorgänge während der Differenzierung der dendritischen Zellen und von Zellen die zu dendritischen Zellen reifen aufgeklärt werden und/ oder mit deren Hilfe Vorgänge aufgeklärt werden, die bei der Aktivierung, Inhibierung oder Modulation des Immunsystems und seiner Immunantwort durch die DC oder ihre Vorläuferzellen beeinflusst werden. Zu der Aufklärung dieser Vorgänge gehören auch die Aufklärung von anderen Einflüssen wie beispielsweise der Einfluß von stimulatorischen Molekülen und/oder ihre zeitliche Wirkung beipielsweise während der Differenzierung der DC und Aktivitätsmodulierung des Immunsystems. Diese Modell-und Testsysteme können beispielsweise in Form von Kits und/oder High-Throughputsystemen verwendet werden. Die einzelnen Testarten und ihre Durchführung sind dem Fachmann bekannt.

Unter einem **Modell- und Testsystem für die Testung von Substanzen die auf das Immunsystem wirken** versteht man

Testsysteme, die als Komponente eine CD124+ und CD116+ Tumorzellinie haben, die bevorzugterweise auch CD34+ ist, und mit deren Hilfe getestet werden kann ob Substanzen einen Einfluß auf das Immunsystem und/oder dessen Konditionierung haben. Darunter fallen unter anderem auch Testsysteme, die der Entwicklung von Immuntherapeutika dienen, beipielsweise die Testung geeigneter Tumorvakzinen und ihrer Formulierungen und solche Testsysteme, die es erlauben den Einfluß von Substanzen, die keine Immuntherapeutika sind, wie beispielsweise chemische Stoffe, Pharmakologische Agenzien, Kosmetika oder ihre Vorstufen, oder Nahrungsmittel oder ihre Komponenten, auf das Immunsystem zu testen. Solche Testsysteme können damit zur Produktentwicklung von beispielsweise Immuntherapeutika und anderen Produkten dienen, die einen Einfluß auf das Immunsystem ausüben können. Diese Modell-und Testsysteme können beispielsweise in Form von Kits und/oder High-Throughputsystemen verwendet werden. Die einzelnen Testarten und ihre Durchführung sind dem Fachmann bekannt.

Zellen, die positiv für CD124 sind tragen den Rezeptor für IL4, die CD116+ den Rezeptor für GM-CSF, und die CD34+, den Marker für haematopoietische Stamm- und Progenitor-Zellen

Weiterhin ist ein Verfahren zur Identifizierung von Peptiden beschrieben, die von den erfindungsgemäßen effektiven Dendritischen Zellen präsentiert werden, umfassend die Schritte
1. (a) Vermehren der erfindungsgemäßen Dendritischen Zellen nach an sich bekannten Methoden, wobei die Dendritischen Zellen unreif sind;
2. (b) Zugeben von Antigenen oder Immunogenen oder Teilen davon oder Zelllysaten, wobei die unreifen Dendritischen Zellen (iDC) zu reifen Dendritischen Zellen (mDC) reifen, und die Antigene oder Immunogene oder Teile davon oder die Zelllysate prozessieren und geeignete Peptide im Kontext von MHC Klasse I oder MHC Klasse II Molekülen präsentieren;
3. (c) Gewinnen der präsentierten Peptide von den Dendritischen Zellen nach an sich bekannten Verfahren; und
4. (d) Identifizieren/Bestimmen der abgelösten Peptide nach an sich bekannten Methoden.

Bevorzugt werden die gewonnenen Peptide durch an sich bekannten Methoden aufgetrennt, wie z.B. mittels Hochdruckflüssigkeitschromatographie (HPLC). Besonders bevorzugt werden die aufgetrennten Peptide massenspektrometrisch identifiziert und am meisten bevorzugt werden die Peptide sequenziert.

Bevorzugt, erlaubt das Verfahren die Validierung der identifizierten/bestimmten Peptide. Mehr bevorzugt wird, dass identifizierte/bestimmte Peptide, die nach dem Verfahren gewonnen werden, synthetisch nach an sich bekannten Methoden hergestellt werden. Am meisten bevorzugt wird, dass die synthetisch hergestellten Peptide zu unreifen und/oder reifen erfindungsgemäßen Dendritischen Zellen zugegeben werden, wobei die Dendritischen Zellen nach an sich bekannten Methoden beladen ("gepulst") werden. In einer Variante wird anstatt des Schrittes (b) folgender Schritt duchgeführt: Beladen von zu mDC gereiften Zellen mit MHC I und/oder MHC II Peptiden. Diesem Schritt kann ein Schritt nach an sich bekannten Methoden zur Entfernung bestehender MHC I und/oder MHC II Peptide vorgeschaltet sein. Bevorzugt handelt es sich dabei um Bibliotheken von MHC I und/oder MHC II Peptiden, die den mDC angeboten werden. Mit der Erfindung konnten überraschenderweise leukämische Zelllinien mit einer bestimmten Eigenschaft gefunden werden, die sich in allen Aspekten wie ein immortalisiertes Equivalent von CD34+ DC Precursor Zellen verhält und für Untersuchungen der DC Biologie, der Testung von Substanzen die das Immunsystem beeinflussen und für Immuntherapeutika geeignet sind. Die bestimmten Eigenschaften der Tumorzelllinien ist eine Positivität für CD124 (IL-4R) und Die myeloide Zelllinie MUTZ-3 ist die Zelllinie, die beim erfindungsgemäßen Verfahren zur Herstellung von effektiven dendritischen Zellen oder Zelllinien verwendet wird. Von MUTZ-3 wurde kürzlich bekannt, dass sie auf Stimulation mit IL-4 und GM-CSF die Expression von CD14 herrunterreguliert. Die Untersuchungen der Erfindung zeigen, dass die im Vergleich mit anderen bekannten und getesteten leukämischen Zelllinien und anderen Tumorzelllinien MUTZ-3-Zellen einzigartig sind in ihrer Fähigkeit, einen unreifen DC-Status zu erlangen. Zudem exprimieren sie nach weiterer Stimulierung den Reifungsmarker CD83, und funktionelle Assays beweisen ihre Fähigkeit zur Antigen-Prozessierung und -Präsentation. Damit ist sie für Immuntherapeutkazwecke geeignet. MUTZ-3 ist die erste humane Leukämiezelllinie, die zur Differenzierung und zur Ausprägung eines unreifen DC-Phänotyps angeregt werden kann und ist als in vitro Modell für Untersuchungen der molekularen und physiologischen Wege, die zur Differenzierung und Reifung DC führen und zur Untersuchung der DC-Biologie und zur Testung von Substanzen, auf einen Einfluß auf das Immunsystem geeignet.

Im Rahmen der Erfindung wird überraschend gezeigt, dass es möglich ist, aus der MUTZ-3 Zelllinie effektive DC zu generieren, die insbesondere als Immuntherapeutika oder als Teil von Immuntherapeutika zur Behandlung von Immunerkrankungen eingesetzt werden können. Kerneigenschaften der Zelllinie ist ihre CD124 und CD116 Positivität
und die Sensitivität gegenüber stimulatorischen Molekülen, wie beispielsweise Zytokinen, während andere untersuchte leukämische Zelllinien, die diese Eigenschaften nicht zeigen, keine effektiven DC im Sinne der Erfindung ergeben. Bevorzugt wird,
dass aus der Zelllinie durch eine sequentielle Stimulation mit stimulatorischen Molekülen DCs unterschiedlicher Aktivierungs- und Effektorstadien gewonnen werden. Die einzelnen Aktivierungs- und Effektorstadien können als effektive DC für die Aktivierunge unterschiedlicher Teile des Immunsystems dienen, und dabei entweder über MHCI Präsentation CD8+ T Zellen aktivieren, über MHCII Präsentation CD4+ T Zellen aktivieren, oder über CD1 NKT Zellen aktivieren. Aktivierte DC werden dabei hauptsächlich für Immuntherapeutika eingesetzt, die für die Behandlung von Infektionskrankheiten und Tumorerkrankungen verwendet werden. Weiterhin können geeignete DC Aktivierungsstadien zur Induktion von Anergien und Toleranzen führen, und eignen sich dazu zur Behandlung von Autoimmunerkrankungen.

Im weiteren ist die Erfindung anhand der humanen myeloiden Zelllinie MUTZ-3 näher beschrieben.
Die humane akute myeloide leukämische Zelllinie MUTZ-3 ist sensitiv gegenüber solchen Zytokinen, die dafür verantwortlich sind, dass in in vivo und in in vitro Modellen aus Monozyten und CD34 positiven Stammzellen DC generiert werden. MUTZ-3 Zellen verhalten sich in allen Eigenschaften wie immortalisierte Äquivalente von CD34 positiven DC-Vorläufer Zellen. Durch Stimulation mit den jeweils geeigneten spezifischen Zytokin-Cocktails entwickeln sie sich zu Zellen mit Phenotypen, die den Phenotypen von z.B. interstitiellen DC oder Langerhans Zellen entsprechen. Durch eine Reifung (Maturation) exprimieren die Zellen CD83. MUTZ-3 besitzt das komplette Spektrum von Antigenprozessierungs- und Präsentierungsprozessen für MHC-abhängige und CD1d-abhängige Präsentation und Aktivierung. Unter geeigneten Bedingungen, z.B. Gabe von Interferon-gamma oder Dexamethason, können sie gezielt einen DC1 Phänotyp oder DC2 Phänotyp annehmen wodurch eine Immunantwort gesteuert werden kann. Dies zeigt, dass MUTZ-3 Zellen eine unlimitierte Quelle für CD34 positive DC Vorläuferzellen (Progenitors) darstellen, die effektiv in der (gezielten) Stimulierung der verschiedenen Immunzellen und somit als effektive DC für die Behandlung von Immunkrankheiten eingesetzt werden können.

Die im Sinne der Erfindung wichtige Komponente für Immuntherapeutika ist die Zelllinie,die selbst effektive DC darstellt oder aus der durch eine Behandlung mit geeigneten stimulatorischen Molekülen effektive DC entstehen. Im Sinne der Erfindung können die effektiven DC mit weiteren Komponenten zu allogenen oder semi-allogenen Immuntherapeutika kombiniert werden, wobei es auch bei Bedarf zu einer weiteren Reifung der Zellen gegebenenfalls durch geeignete stimulatorische Moleküle kommen kann. Im Beispiel 1 ist dies für MUTZ-3 jeweils für MHCI, MHCII und CD1 vermittelte Aktivierung beschrieben. Darauf ist die Erfindung jedoch nicht beschränkt, sondern umfasst alle therapeutischen oder prophylaktischen Anwendungsgebiete, bei der DC Zellen eingesetzt werden können.

Hierunter fallen beispielsweise Tumortherapeutika. Diese können beispielsweise hergestellt werden, indem die allogenen der semi-allogenen effektiven DC beipielsweise mit Tumorantigenen nach an sich bekannten Methoden gepulst und dem Patienten verabreicht werden. Tumorantigene können dabei einzelne oder mehrere definierte Moleküle wie beispielsweise Peptide, Glykopeptide, Proteine, Glykoproteine, Glykolipide sein, die synthetisiert, gereinigt oder in Form von Zelllysaten verwendet werden; ein weiteres Beispiel ist die Transfektion der effektiven DC mit RNA, DNA oder viralen Vektoren, die Tumorantigene oder Teile davon kodieren; ein weiteres Beispiel ist die Antigenbeladung von effektiven DC durch Inkubation mit apoptotischen und/oder nekrotischen Tumorzellen, oder mit hitzeschockbehandelte Zellen; ein weiteres Beispiel ist die Fusion mit Tumorzellen.Eine klinische Anwendung solche im Rahmen der Erfindung hergestellte DC erfolgt in Form allogener oder semi-allogener DC prophylaktisch oder als kurative Therapie, z.B. um Tumoren zu therapieren oder nach Entfernung solcher Tumoren, z.B durch Chirurgie, als Adjuvanztherapie zur Behandlung der minimalen residualen Erkrankung einschließlich Metastasen zu bekämpfen oder um die Bildung von Metastasen bzw. Mikrometastasen zu verhindern.

Eine Reihe von Immunisierungs-Strategien sind möglich, u.a. die intranodale, intratumorale, intradermale, intramuskuläre, subkutane, intraperitoneale or mukosale Applikation von DC mit oder ohne zusätzliche Immunostimulanzien wie, z.B. Zytokine, Chemokine oder andere immunostimulatorische bzw. immunomodulatorische Substanzen. Dabei können die erfindungsgemäß hergestellten DC auch Teil komplexerer Immunisierungsschemata sein bei der beispielsweise weitere Komponenten zeitgleich oder zeitversetzt gegeben werden.

Obwohl sich die aus MUTZ-3 abgeleiteten DC nach Differenzierung nicht mehr teilen, kann es nicht ausgeschlossen werden, daß DC die aus anderen Leukämie Zellen oder Linien hergestellt werden können sich weiterhin teilen. Daher ist eine bevorzugte Variante die Bestrahlung solcher Antigen-beladenen DC, ihre Behandlung mit Mitomycin C, oder eine andere Maßnahme, die eine Teilung der Zellen in vivo verhindert. Eine Alternative wäre beispielsweise die Inkorporation eines sogennaten Suizid-Genes, wie z.B. das HSV Thymidine Kinase (TK) Gens, das es erlaubt solche HSV TK-tragende Zellen durch Gancyclovir selektiv abzutöten.

Hierin ist auch die Herstellung von Zelllinien beschrieben, die zu effektiven DC gereift werden können. Das erfindungsgemäße Verfahren beinhaltet die Isolierung von CD34+, CD124+ und CD116+ Zellen nach an sich bekannten Verfahren aus humanem Material bevorzugt Leukämiepatienten. Die Zellen können beispielsweise aus dem peripheren Blut oder Knochenmark von Leukämiepatienten durch Anreicherung von Zellen, die CD34+, CD124+ und CD116+ sind, mit Hilfe von Magnetbeads, die Antikörpertragen, die CD34+, CD124+ und CD116+, sequentiell gewonnen werden. Alternativ können CD34+, CD124+ und CD116+ Zellen durch Zellsorting mittels Durchflußzytometrie und CD34-, CD124- und CD116-spezifischen Antikörpern gewonnen werden.
Weiterhin beschrieben ist ein Verfahren zur Herstellung eines Arzneimittels, umfassend die Schritte der erfindungsgemäßen Verfahren und weiterhin umfassend den Schritt der Formulierung des Arzneimittels in pharmazeutisch verträglicher Form, wobei das Arzneimittel gegebenenfalls zusätzlich mit einem Adjuvans als Wirkstoffverstärker kombiniert wird

Unter dem Begriff "Arzneimittel" sind erfindungsgemäß Stoffe und Zubereitungen aus Stoffen definiert, die dazu bestimmt sind, durch Anwendung am oder im menschlichen Körper Krankheiten, Leiden, Körperschäden oder krankhafte Beschwerden zu heilen, zu lindern oder zu verhüten. Während des erfindungegemäßen Herstellungsverfahrens können den mit den erfindungsgemäßen Verfahren identifizierten Verbindungen medizinische und/oder pharmazeutisch-technische Hilfsstoffe zugesetzt werden. Medizinische Hilfsstoffe sind erfindungsgemäß solche Stoffe, die zur Produktion (als aktive Ingredienzien) von Arzneimitteln in einem erfindungsgemäßen Verfahren eingesetzt werden. Pharmazeutischtechnische Hilfsstoffe dienen lediglich der geeigneten Formulierung des Arzneimittels und können sogar, sofern sie nur während des Verfahrens benötigt werden, anschließend entfernt werden oder können als pharmazeutisch verträgliche Träger Teil des Arzneimittels sein. Beispiele für pharmazeutisch verträgliche Träger sind nachstehend aufgeführt.
Die Arzneimittelformulierung erfolgt gegebenenfalls in Kombination mit einem pharmazeutisch verträglichen Träger und/oder Verdünnungsmittel.
Beispiele für geeignete pharmazeutisch verträgliche Träger sind dem Fachmann bekannt und umfassen Phosphat-gepufferte Kochsalzlösungen, Wasser, Emulsionen wie z.B. Öl/Wasser-Emulsionen, verschiedene Arten von Detergenzien, sterile Lösungen, etc.
Arzneimittel, die solche Träger umfassen, können mittels bekannter konventionelle Methoden formuliert werden. Bevorzugt werden Applikationsrouten, bei denen die erfinderischen effektiven dendritischen Zellen in einer pharmakologischen Formulierung an Stellen im Körper gebracht werden, wo sie ihre Aufgabe am besten erfüllen können. Solche Stellen und Applikationsformen sind dem Fachmann bekannt, beispielsweise intravenös, intraperitoneal, subkutan, intramuskulär, lokal oder intrademal, bevorzugt dabei sond beispielsweise intra nodal, intra dermal, subcutan, intrarektal, intravenös oder lokal. Eine geeignete Applikationsroute kann abhängig von der Erkrankung unterschiedlich geeignet sein.

Beispielsweise zielt eine Applikation von effektiven dendritischen Zellen für die Therapie von Autoimmunerkrankungen auf eine Toleranz des Immunsystems ab, während eine geeignete Applikationsroute für die Behandlung oder Prophylaxe von Tumor- oder Infektionserkrankungen eine Aktivierung des Immunsystems unterstützen soll. Eine geeignete Administrationsroute kann vom Fachmann mit bekannten Methoden bestimmt werden. Die Arzneimittel können einem Individuum in iner geeigneten Dosis verabreicht werden, eine Dosis umfasst 100 bis 10¹² effektive dendritische Zellen, bevorzugt 10⁵ bis 10¹⁰. Die effektiven dendritischen Zellen wurden dabei mit einer geeigneten Form und Menge an Antigenen beladen die ebenfalls je nach Einsatz verändert sein kann. Eins Dosis wird bevorzugt einmal die Woche, bis hinzu größeren Abständen verabreicht wie beispielsweise einem Monat, 3 Monaten, einem Jahr oder noch längere Abstände. Kürzere Intervalle können ebenfalls geeignet sein, wie beispielsweise täglich. Dabei kann der Fachmann den geeigneten zeitlichen Abstand und Dosis bestimmen. Hierfür kann er bevorzugt Methoden zum Immunomontoring verwenden und die Dosen entsprechend anpassen. Entsprechende Methoden sind dem Fachmann bekannt und werden zum Teil in den Beispiele beschrieben.
Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberflache, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

In einer bevorzugten Ausführungsform können die effektiven dendritischen Zellen der Erfindung mit einer Reihe von Antigenen beladen werden.
In einer weiteren bevorzugten Ausführungsform werden Dosen mit effektiven dendritischen Zellen beladen mit geeigneten Antigenen mit Dosen kombiniert, die die oder einzelne Antigene oder Teile davon direkt in geeigneten Formulierungen ohne ex vivo Beladung der dendritischen Zellen umfassen. Dies hat den Vorteil, dass semi-allogene ex vivo beladenen erfindungsgemäße dendritische Zellen die Immunantwort stark induzieren, wobei sie von der Alloresponse als eine Art Danger Signal unterstützt werden und verbunden sind mit einer teilweise spezifischen Immunisierung durch die Präsentation überlappender MHC Moleküle, kombiniert werden mit einer Immunantwort, die die autologen dendritischen Zellen in vivo ansteuert. Eine solche Kombination ist besonders geeignet um Toleranzen und Anergien zu brechen.
In einer bevorzugten Ausführungsform werden erfindungsgemäße unreife effektive dendritische Zellen (iDC-Form), beladen mit entsprechenden Antigen, zur Behandlung von Autoimmunerkrankungen eingesetzt. Dabei werden in einer weiter bevorzugten Ausführungsform die Zellen in der iDC Form transient oder stabil arretiert, wobei an sich dem Fachmann bekannte Methoden eingestezt werden, beispielsweise eine Arretierung durch gentechnische Veränderung. In einer weiteren bevorzugten Ausführungsform werden die Zellen nach Beladung in der unreifen Form (iDC) oder reifen Form (mDC) gegebenenfalls weiter gereift und als beladene effektive dendritische Zellen (mDC) zur Behandlung oder Prophylaxe von Tumor- oder Infektionserkarnkungen eingesetzt.
Ein erfindungsgemäßes Arzneimittel umfasst eine pharmakologische Substanz, die Dendritische Zellen in einer geeigneten Lösung oder Verabreichungsform enthält. Diese können entweder alleine oder in Kombination mit einem oder mehreren Adjuvatien oder einem anderen geeigneten Stoff zur Wirkungsverstärkung verabreicht werden. Als bevorzugte Adjuvantien werden QS-21, GPI-0100 oder andere Saponine, Wasser-Öl Emulsionen wie beispielsweise Montanide Adjuvantien, Polylysin, Polyargininverbidnungen, DNA-Verbindungen wie beispielsweise CpG, Detox, Bakterielle Vakzinen wie beispielsweise Typhusvakzine oder BCG-Vakzinen verwendet und in einer entsprechend geeigneten Weise nach an sich bekannten Methoden mit den erfindungsgemäßen Dendritischen Zellen gemischt.
Eine bevorzugte Form der Adjuvantien sind costimulatorische Faktoren, Cytokine und/oder Wachstumsfaktoren wie beispielsweise GM-CSF oder IL-2 oder IL-12. Diese können auch in genetischer Form in die Zellen der erfindungsgemäßen Zelllinien, bevorzugt stabil, eingebracht werden.
Die Verwendung des Arzneimittels ist zur Prophylaxe und/oder Behandlung von Krebserkrankungen, Tumoren, Infektionen und/oder Autoimmunerkrankungen geeignet.

Bevorzugt ist die Krebserkrankung oder der Tumor, der/die behandelt oder verhindert wird ausgewählt aus der Gruppe von Krebserkrankungen oder Tumorerkrankungen des Kopfes und Genicks, der Lunge, des Mediastinums, Gastrointestinaltrakts, des Geschlechts/Harnapparatsystems, des gynäkologischen Systems, der Brust, des endokrinen Systems, der Haut, Krebserkrankungen oder Tumorerkrankungen der Kindheit, Primärtumoren, metastatischem Krebs, Weichteil- oder Knochensarkomen, einem Melanom, einem Neoplasma des zentralen Nervensystems, Lymphomen, Leukämien, paraneoplastischem Syndrom, peritonealer Carcinomastose und/oder Malignität, die zu immunsupprimierter Malignität verwandt ist.

Die Infektion, die mit dem erfindungsgemäßen Arzneimittel behandelt oder verhindert wird, ist ausgewählt aus bakteriellen Infektionen, viralen Infektionen, pilzlichen Infektionen, Infektionen mit Protozoen und/oder Infektionen mit Helminthen. Bevorzugt ist die bakterielle, virale, pilzliche Infektion, Infektion mit Protozoen und/oder Infektion mit Helminthen, die behandelt oder verhindert wird, ausgewählt aus Infektionen wie Sepsis oder septischer Schock, Fiber unbekannten Ursprungs, infektiöse Endokarditis, intraabdominale Infektionen und Abzesse, akuten Infektionen, Durchfallerkrankungen, bakterielle Lebensmittelvergiftungen, sexuell übertragbaren Infektionen, entzündliche Beckeninfektionen, Harnwegsinfektionen, Pyelonephritis, Osteomyelitis, Infektionen der Haut, Muskeln oder Weichteilen, Infektionen durch Injektion von Drogen, Infektionen durch Bisse, Kratzer oder Verbrennungen, Infektionen bei Transplantatempfängern, Hospitalismus-Infektionen und/oder intravaskuläre Infektionen durch Geräte. In einer mehr bevorzugten Ausführungsform wird die Infektion, die verhindert oder behandelt wird, ausgewählt aus bakteriellen Infektionen wie Pneumococcen-Infektionen, Staphylococcen-Infektionen, Streptococcen-Infektionen, Enterococcen-Infektionen, Diphterie, andere Corynebakterien-Infektionen, Anthrax, Listeria monocytogenes-infektionen, Tetanus, Botulismus, Gasbrand, Antibiotika-assoziierter Colitis, anderen Clostridien-Infektionen, Meningococcen-Infektionen, Gonococcen-Infektionen, Moraxella (branhamella) catarrhalis-Infektionen, anderen Moraxella species-Infektionen, Klingella-Infektionen, Haemophilus influenzae-Infektionen, anderen Haemophilus species-Infektionen, Infektionen durch die HACEK Gruppe, Infektionen durch andere Gram-negative Bazillen, Legionella-Infektionen, Pertussis, Infektionen durch Gram-negative Entero-Bazillen, Helicobacter-Infektionen, Infektionen durch Pseudomonaden und verwandte Organismen, Salmonellose, Shigellose, Infektionen durch Campylobacter und verwandte Species, Cholera, Vibrio, Brucellose, Tularaemie, Pest, anderen Yersinien-Infektionen, Bartonella-Infektionen, einschließlich Infektionen durch Katzenkratzer, Donovania (Granuloma Inguinale), Nocardiose, Actinomykose, Infektionen durch gemischte anaerobe Organismen, Tuberkolose, Lepra, Infektionen durch nicht-Tuberkelbakterien, Syphilis, endemische Treponematose, Leptospirose, Rückfall-Fieber, Lyme Borreliose, Infektionen durch Rockettsien, Mycoplasmen oder Chlamydien, virale Infektionen wie Herpes simplex Virus-Infektionen, Varizella zoster-Infektionen, Epstein Barr-Virus-Infektionen, einschließlich Mononucleose, Cytomegalovirus-Infektionen, humane Herpesvirus Typ 6,7 oder 8-Infektionen, Pockenvirus-Infektionen, Vaccinia-Infektionen, andere Pockenviren-Infektionen, Parvovirus-infektionen, humane Papillomavirusinfektionen, virale Atemwegsinfektionen, Influenza, virale Gastroenteritis, Enterovirus-Infektionen, Reovirus-Infektionen, Masern, Röteln, Mumps, Rabies-Virus-infektionen, anderen Rhabdovirus-Infektionen, Infektionen hervorgerufen durch Nager- und/oder Arthropodenviren, Infektionen mit Marburg- und/oder Ebolaviren, pilzlichen Infektionen wie Histoplasmose, Coccidioidomykosis, Blastomykose, Cryptococcose, Candidiosis, Aspergillose, Mucormycosis, verschiedene Mykosen, Prothotheca-Infektionen, Pneumocystis carinii-Infektionen, Infektionen mit Protozoen, wie Amöbenbefall, Infektionen mit freilebenden Amöben, Malaria, Infektionen durch Parasiten von roten Blutzellen, Leishmaniosis, Trypanose, Toxoplasma-Infektionen, Intestinalinfektionen durch Protozoen, Trichomonadenkolpitis, Infektionen mit Helminthen, wie Trichinose, Infektionen mit anderen Gewebe-Nematoden, Infektionen mit intestinalen Nematoden, Filariose, Infektionen wie Loiasis, Onchozerkose oder Dracontiasis, Schistosomie, Trematoden-Infektionen oder Cestoden-Infektionen.
Die Autoimmunkrankheit, die mit dem erfindungsgemäßen Arzneimittel behandelt oder verhindert wird, ist ausgewählt aus Autoimmunkrankheiten wie allergische Enzephalomyelitis, autoimmune haemolytische Anämie, autoimmune Thyreoditis (Hashimoto' Syndrom), autoimmune männliche Sterilität, pemphigoide Blasensucht, Bauchhöhlenkrankheit, Basedow Krankheit, Goodpasture-Syndrom, idiopathische thrombocytopenische Purpura, Insulin-resistenter Diabetis mellitus, Myastenia gravis, perniziöse Anämie, Pemphigus vulgaris, Polyarteriitis nodosa, primäre Gallenzirrhose, Reiter' Syndrom, rheumatisches Fieber, Sarcoidosis, Sjögren' Syndrom, systematischer Lupus Erythematodes, sympathische Ophthalmia, Multiple Sklerose, und/oder virale Myocarditis durch Cocksakie B Virus Antwort. Weiterhin beschrieben ist. ein Verfahren zur Herstellung eines Arzneimittels umfassend die erfindungsgemäßen Verfahren, wobei das Arzneimittel Dendritische Zellen enthält, die nach an sich bekannten Methoden mit Antigenen beladen werden oder mit entsprechenden Zellen fusioniert werden. Die Dendritischen Zellen des Arzneimittels werden nach den an sich für autologe Dendritische Zelltherapien bekannten Verfahren mit einem geeigneten pharmazeutischen Träger formuliert. Das so gewonnene Arzneitmittel kann nach an sich bekannten Methoden verabreicht werden. Die dendritischen Zellen des Arzneimittels nehmen Antigene auf, prozessieren sie und präsentieren Fragmente davon auf ihrer Oberfläche im Kontext mit MHC Molekülen und costimulatorischen Molekülen. Nach einer weiteren Reifung nach an sich bekannten Methoden werden die Zellen in einer geeigneten Formulierung am Menschen angewandt. Ein weiteres Beispiel ist die Beladung reifer dendritischer Zellen nach an sich bekannten Methoden des "Pulsing". Bei den dendritischen Zellen handelt es sich um autologe, allogene oder semi allogen dendritische Zellen beziehungsweise deren Vorläuferzellen oder Zellen aus Zelllinien, die die funktionellen Eigenschaften von dendritischen Zellen besitzen, die ex vivo nach an sich bekannten Methoden entsprechend geeignete Behandlung zur Entwicklung und Reifung bekommen. In einer bevorzugten Weise werden die Vorläuferzellen,wenn notwendig durch Gabe von geeigneten Faktoren, beispielsweise costimulatorischen Faktoren, Cytokinen und/oder Wachstumsfaktoren, wie beispielsweise IL-4 und GM-CSF zu Zellen gereift, die funktionell und phänotypisch den iDC ähnlich sind. Diese Zellen werden mit geeigneten, Antigenen beladen bei Bedarf weiter gereift. Die resultierenden beladenen effektiven dendritischen Zellen (mDC), sind Zellen, die reifen beladenen dendritischen Zellen phänotypisch und funktionell ähnlich sind werden bevorzugt zur Prophylaxe oder Therapuie von Tumor- oder Infektionserkrankungen eingesetzt. Alternativ können die effektiven dendritische Zellen erst als mDC beladen werden, wie beispielsweise für definierte MHC-Kalsse Peptide als Antigene. Alternativ werden Zellen in den unterschiedlichen Vorläufer-, Differenzierungs- und/oder Reifungsstadien mit DNA oder RNA von Antigenen, costimulatorischen Molekülen und/oder Immunogenen nach an sich bekannten gentechnologischen Methoden transfiziert werden. Bevorzugt sind dies stabile Transformationen von den Zellen, die sich am besten teilen lassen,bevorzugt vor dem Vorläuferstadium vor der Differenzierung. Alternativ werden geeignete Stadien der erfindungsgemäßen dendritischen Zellen, als Vorläuferzelle, als unreife Zelle oder als reife Zelle mit anderen Zellen nach an sich bekannten Methoden fusioniert und gegebenfalls weiter differenziert und oder gereift. Für die Behandlung oder Prophylaxe von Autoimmunerkrankungen werden bevorzugt die beladenen Zellen im unreifen Stadium verwendet, wie weiter oben detaillierter beschrieben wird.

Die Erfindung wird durch folgendes Beispiel näher erläutert, ohne sie auf dieses Beispiel einzuschränken:

### Beispiel 1:

MUTZ-3, eine humane CD34+, CD124+, CD116+ Zelllinie zur Herstellung von effektiven DC durch eine Zytokin-induzierte Differenzierung von dendritischen Zellen aus CD34+ Precursor Zellen und die Verwendung der effektiven DC zur Induktion funktioneller T-Zell Subsets für die Herstellung von Immuntherapeutika

### Material und Methoden

### Antikörper und Reagenzien

Für die Untersuchungen wurden verwendet:
PE-markierte monoklonale Antikörper (mAK) gegen CD40, CD34 und TCR Valpha 24 von Coulter Immunotech (Marseilles, France), gegen CD1a, CD54, CD83 und CD86 von Pharmingen (San Diego, CA) und gegen CD80 von Becton-Dickinson (San Jose, CA)
FITC-markierte mAK gegen HLA-DR, TCR Vβ 11 und CD14 von Becton-Dickinson, gegen CD116 (GM-CSF-Rezeptor) von Pharmingen.
Die CD1d-Expression wurde bestimmt mittels eines murinen mAK gegen CD1d (Mab CD1d27) (Spada et al. 1998, J Exp Med 188(8): 1529-1534) gefolgt von einem FITC-markierten anti-Maus-IgG1 mAK (Pharmingen). Die Isotyp-Kontrolle Maus-IgG1 stammt von Organon Technika-Cappel (Malvern, PA)., FITC-und PE-markierte Simultest-Isotyp-Kontrollen von Becton-Dickinson. Langerin-Expression wurde durch Färbung mit dem mAK DDCM4 gefolgt von einem FITC-markierten anti-Maus-mAK nachgewiesen. Die Blockierung der Antigen-Präsentation über CD1d erfolgte mit dem AK CD1d51 (Spada et al. 1998, J Exp Med 188(8): 1529-1534).

### Zellkulturen

Die Zytokin-abhängige, humane myelomonozytische Leukämie-Zellinie MUTZ-3 wurde in MEM-alpha mit Ribonukleosiden und Desoxiribonukleosiden (Gibco, Paisley, UK), Hitze-inaktiviertem FKS, Penicillin/Streptomycin und 10 % konditioniertem Medium der humanen Blasenkarzinom-Zellinie 5637 (Quentmeier 1996, Leuk Res (4):343-350) kultiviert. Die Zellen wurden in 6-Loch-Platten (Costar, Cambridge, MA) bei 37°Cund 5% CO₂ kultiviert und zweimal wöchentlich passagiert. Die von einer akkuten monozytischen Leukämie abgeleitete Zellinie THP-1, die von einer akkuten myelogenen Leukämie abgeleitete Zellinie KG-1, die chronisch myeloische Leukämielinie K562, die von einer promyelozytischen Leukämie abgeleitete Zellinie HL-60 und die Makrophagen-ähnliche histozytische Lymphomlinie U937 wurden von der Amerikanischen Type Culture Collection (ATCC, Rockville, MD) erhalten. Diese Zellinien wurden in IMDM oder RPMI-1640 mit Hitze-inaktiviertem FKS, Penicillin/Streptomycin, 2M L-Glutamin und beta-Mercaptoethanol kultiviert und zweimal wöchentlich in 80 cm² Gewebekulturflaschen (Costar) passagiert.

### Generierung von unreifen (iDC) und reifen DC-ähnlichen (mDC) Zellen aus Leukämiezellinien

Die Induktion eines DC-ähnlichen Phänotyps in Leukämiezellinien wurde wie folgt erreicht:
Die Zellen wurden gewaschen und mit einer Zelldichte von 1x10⁵/ml (in einem Volumen von 3ml) in 24-Loch-Platten eingesät und für 7 Tage mit GM-CSF (100ng/ml Novartis/Schering-Plough, Arnhem, NL), IL-4 (1000U/ml CLB) und niedrigdosiertem TNFalpha (2,5 ng/ml, CLB, Amsterdam, NL) inkubiert. Am Tag 7 wurde die Reifung induziert durch Gabe von entweder TNFalpha (75ng/ml)oder LPS (100ng/ml, Sigma). Für die Herstellung von LC-ähnlichen Zellen wurden die MUTZ-3-Zellen für 9 Tage in GM-CSF und niedrigdosiertem TNFα kultuviert. Die Zellen wurden dann mit oder ohne TGFβ1 (1ng/ml, R&D Systems, Abingdon, Oxon UK) und niedrigdosiertem TNFα für weitere 7 Tage inkubiert, wobei das Kulturmedium am Tag 2 erneuert wurde. Die dadurch erhaltenen unreifen DC (iDC) wurden auf die Expression von CD1a und Langerin untersucht.

### Durchflußzytometrie

Die kultivierten Zellen wurden gewaschen und mit einer Zellzahl von 5x10⁴ bis 1x10⁵ in 25 microl eiskaltem FACS-Puffer (PBS pH 7,5, 0,1%BSA, 0,2%Natriumacid) resuspendiert. Die spezifischen und die fluoreszenzmarkierten mAK bzw. die zugehörigen Isotyp-Kontrollen wurden zugegeben und die Zellen für 30 min bei 4°C inkubiert. Die Zellen wurden einmal gewaschen und in 250 microl FACS-Puffer resuspendiert. Die markierten Zellen wurden an einem FACStar (Becton Dickinson) unter Verwendung der CellQuest-Software analysiert.

### Allogene Gemischte-Lymphozyten-Reaktion (mixed lymphocyte reaction, MLR)

Allogene, nichtadhärente PBL wurden mittels Gradientenzentrifugation über Hypaque Lymphoprep (Nycomed, Oslo, Schweden) aus dem peripheren Blut gesunder Spender isoliert. Die Zellen wurden in einer Konzentration von 5x10⁴ Zellen/Loch in Rundboden-Mikrotiterplatten eingesät und mit einer Verdünnungsreihe an MUTZ-3 DC in 200 microl Kulturmedium für 5 Tage inkubiert. Die T-Zell-Proliferation wurde nach einen 5h-Puls mit ³H-Thymidin (0,4 microCi/Loch, Amersham, Aylesbury, UK) bestimmt (Standardmethoden).

### Induktion von IL-12/p70- und IL-10-Sekretion durch reife MUTZ-3-DC (MUTZ-3 mDC)

Die MUTZ-3iDC wurden gewaschen und mit einer Zellzahl von 1x10⁵ in MEM alpha (Zusätze siehe oben) in 48-Loch-Platten eingesät. Unreife MUTZ-3-DC (MUTZ-3 iDC) wurden durch Behandlung mit TNFα in Kombination mit entweder IFNgamma (1000 U/ml, Biosource, Camarillo, CA) oder Dexamethason (1micromol/l, Sigma) (Inkubationszeit 48h) und anschließende Stimulation mit bestrahlten Zellen einer cD40-Ligand-transfizierten J558-zellinie (J558-CD40L, 1x10⁵ Zellen/Loch) zur Reifung gebracht. Die Konzentrationen der sekretierten Zytokine IL-10 und IL-12 (p70-Untereinheit) wurden mittels ELISA bestimmt.

### Induktion von CD8⁺-T-Zellen mit einer Spezifität für Influenza-Matrix-Proteine

MUTZ-3-DC wurden mit 100pfu/zelle eines rekombinanten Adenoviruses infiziert. Dieser Adenovirus kodiert das M1-Matrix-Proteingen des Haeminfluenzavirus (RAd128). Für die RAd128-Infektion wurden die DC mit serumfreiem Medium gewaschen und mit Lipofectamin (100pfu/Zelle, 1,7microg/l x 10⁸ pfu) inkubiert. Nach 2h wurden die Zellen mit komplettem Medium gewaschen und dann über Nacht bei 37°C und 5%CO₂ inkubiert. Andere MUTZ-3-DC wurden mit dem HLA-A2.1-bindenden M1-abgeleiteten Peptid M1₅₈₋₆₆ (50µg/ml) über Nacht bei 37°C zusammen mit beta-Mikroglobulin (2,5microg/ml) in serumfreiem Medium beladen. CD8⁺-T-Zellen (Responder) wurden mittels eines CD8-T-Zell-MACS-Isolationskits (Miltenyi Biotec) aus HLA-A2+ PBMC isoliert. Antigen-beladene (Virus oder Peptid) MUTZ-3-DC (Stimulator) wurden mit einem Responder:Stimulator-Verhältnis von 5:1 eingesetzt in komplettem IMDM-Medium mit 10% gepooltem Humanserum (CLB) und 5ng/ml IL-7 (R&D Systems). Nach einer Woche wurde die T-Zellen in einem IFNgamma-ELISPOT-Assay auf Spezifität untersucht. Dazu wurden bestrahlte T2-Zellen, die mit dem HLA-A2.1-bindenden, M1-abgeleiteten Peptid M1₅₈₋₆₆ oder als Kontrolle mit dem HLA-A2.1 bindenden HPV16-E7-abgeleiteten Peptid (E7₁₁₋₂₀) beladen wurden, verwendet. Die Zellen wurden über Nacht bei 37°C mit dem peptid (50microg/ml) und beta-Mikroglobulin (2,5microg/ml) in serumfreiem Medium beladen.

### Induktion von CD8⁺-T-Zellen mit einer Spezifität für das Melanomassoziiertes Antigen, MART-1

MUTZ-3-DC wurden mit dem HLA-A2.1-bindenden MART-1-abgeleiteten Peptid (ELAGIGILTV) (10microg/ml) 4 Stunden bei 37°C in serumfreiem AIM-V-Medium (Gibco) beladen. CD8⁺-T-Zellen (Responder) wurden mittels eines CD8-T-Zell-MACS-Isolationskits (Miltenyi Biotec) aus HLA-A2+ PBMC isoliert. Mit MART-1-peptidbeladene MUTZ-3-DC (Stimulator) wurden mit einem Responder:Stimulator-Verhältnis von 10:1 in serumfreiem AIM-V-Medium (Gibco) eingesetzt. Nach einer Woche wurden die T-Zellen in einem IFNgamma-ELISPOT-Assay auf Spezifität untersucht. Dazu wurden bestrahlte T2-Zellen, die mit dem HLA-A2.1-bindenden MART-1 Peptid (ELAGIGILTV) oder als Kontrolle mit dem HLA-A2.1-bindenden, CEA-abgeleiteten Peptid CEA.78 (IMIGVLVGV) beladen wurden, verwendet. Die Zellen wurden 4 Stunden bei 37°C mit dem Peptid (1 microg/ml) in serumfreiem AIM-V-Medium (Gibco) beladen.

### Induktion von CD8⁺-T-Zellen mit Spezifitäten für die TumorAntigene MUC-1 und Asialoglykophorin durch Stimulierung mit Tumorzelllysaten.

Die Tumorzelllysate wurden entweder aus Tumorzelllinien oder aus Primärmaterial hergestellt:, a) Zelllysate aus Tumorzelllinien wurden mit Hilfe von 4 Runden abwechselndem Frieren in flüssigem Stickstoff und anschließendem Tauen nach an sich bekannten Methoden hergestellt. b) Zelllysate aus solidem Tumorprimärmaterial wurden wie folgt hergestellt: Solide Tumoren wurden mit Hilfe der Triple-Enzym Methode behandelt und dadurch Einzelzellsuspensionen hergestellt. Diese Methode ist dem Fachmann bekannt und wird häufig in verschiedenen Varianten in den meisten Tumorpathologie-/ Immunologielabors angewandt. Nach der chirurgischen Entfernung des Tumors werden alle weiteren Schritte unter aseptischen Bedingungen durchgeführt. Der Tumor wurde in etwa 5 kubikmillimeter große Stücke zerteiltund in ein Gefäß mit sterilem Triple-Enzym-Medium (0,1% Kollagenase, 0,002% Desoxyribonuklease, 0,01% Hyaluronidase in dem Puffer "Hank's buffered saline", HBSS) gegeben. Dies wurde über Nacht bei Raumtemperatur mit einem magnetrührer gerührt bis die soliden Gewebsstücke sich aufgelöst haben. Anschließend wurde über einen Maschenfilter (coarse wire grid) die unverdauten gewebsstücke abgetrennt und die verbliebenen Zellen nach sorgfältigem Waschen in HBSS mit einem Ficoll Gradienten zentrifugiert, um Monozyten und Lymphozyten von der Tumorzellsuspension zu trennen. Die Tumorzellen wurden anschließend mit Hilfe von 4 Runden abwechselndem Frieren in flüssigem Stickstoff und anschließendem Tauen lysiert.

MUTZ-3-DC wurden mit einem Tumorzelllysat über Nacht bei 37°C in serumfreiem AIM-V-Medium (Gibco) beladen. CD8⁺-T-Zellen (Responder) wurden mittels eines CD8-T-Zell-MACS-Isolationskits (Miltenyi Biotec) aus HLA-A2+ PBMC isoliert. Mit Tumorzelllysatbeladene MUTZ-3-DC (Stimulator) wurde mit einem Responder:Stimulator-Verhältnis von 10:1 in serumfreiem AIM-V-Medium (Gibco) eingesetzt. Nach einer Woche wurde die T-Zellen in einem IFNgamma-ELISPOT-Assay auf Spezifität untersucht. Dazu wurden Antigen-beladene MUTZ-3-DC oder Peptid-beladene T2-Zellen verwendet. MUTZ-3-DC wurden über Nacht bei 37°C in serumfreiem AIM-V-Medium (Gibco) mit einem Tumorzelllysat oder mit Asialoglykophorin (Protein) beladen. T2-Zellen wurden mit dem HLA-A2.1-bindenden MUC1-Peptid MUC-1.2(LLLLTVLTV) 4 Stunden (1 microg/ml) in serumfreiem AIM-V-Medium (Gibco) beladen.

### IFNgamma-ELISPOT-Assay

Multiscreen-96-Loch-Filtrationsplatten (Millipore, Molsheim, Frankreich) wurden für 3h bei Raumtemperatur (RT) oder über Nacht bei 4°C mit dem mAK 1-D1K (50microl, 15microg/ml) in filtriertem PBS (Mabtech, Nacka, Schweden) beschichtet. Die Platten wurden 6-mal mit serumfreiem Medium gewaschen und anschließend mit filtriertem kompletten Medium mit 10% FKS für 0,5-1h bei RT blockiert. Anschließend wurden 7,5x10³ bis 1x10⁵ Effektorzellen/Loch mit 1x 10⁴ Zielzellen über Nacht bei 37°C, 5% CO₂ inkubiert. Die Zellen wurden verworfen und die Platten 6-mal mit filtriertem PBS/0,05%Tween 20 gewaschen. Zu jedem Loch wurden dann 50microl mAK 7-B6-1 (1microg/ml in filtriertem PBS) hinzugefügt und die Platten für 2-4h bei RT stehengelassen: Nach 6 Waschschritten mit filtriertem PBS/0;05%Tween 20 wurden 50microl/Loch Streptavidingekoppelte Alkalische Phosphatase (1:1000 verdünnt in PBS) zugegeben und die Platten für 1-2h bei RT inkubiert. Nach 6 weiteren Waschschritten mit filtriertem PBS/0,05%Tween 20 wurden 50microl Alkalische-Phosphatase-Reagenz (AP conjugate substrate kit , Biorad, Herkules, CA) zugesetzt und 15 min bis 1h stehengelassen, bis sich Farbpunkte entwickelt haben. Die Reaktion wurde mit Leitungswasser gestoppt und die Farbpunkte von 2 unabhängigen Personen ausgezählt.

### Aktivierung von Tetanus-Toxoid(TT)-spezifischen T-Zellen

PBMC von Spendern mit partieller HLA-Übereinstimmung (HLA-DR11, HLA-DQ7, HLA-B44 und HLA-A2 exprimierend) wurden ausgewählt und die CD4⁺-PBL mittels MiniMACS-Separationssäulen (Miltenyi Biotec) isoliert. Nach 1,5h Adhärenz an die Plastikoberfläche, um kontaminierende APC zu entfernen, wurden die Zellen mit einer Verdünnungsreihe TT-gepulster, unreifer MUTZ-3-DC (50mg/ml, Bilthoven, NL, 12h in serumfreiem Medium) in 200microl Medium für 7 d bei 37°C , 5% CO₂ inkubiert. Die T-Zell-Proliferation wurde nach einem 5h-Puls mit ³H-Thymidin (0,4 microCi/Loch, Amersham, Aylesbury, UK) bestimmt (Standardmethoden).

### Präsentation von α-Galactosylceramid für Vαc24⁺/Vβ11⁺-NKT-Zellen

Valpha24⁺-T-Zellen, einschließlich Valpha24⁺/Vbetall-⁺-NKT-Zellen, wurden durch Positivselektion aus PBL gewonnen mittels autoMACS (Miltenyi Biotec). Die aufgereinigten NKT-Zellen wurden dann für 7 Tage mit unreifen oder reifen MUTZ-3-DC, die mit DMSO (Vehikelkontrolle) oder 100ng/ml alpha-Galactosylceramid (alpha-GalCer, Pharmaceutical Research Laboratory, Kirin Brewery, Japan) gepulst worden waren, kokultiviert unter der Zugabe von 10ng/ml rekombinantem humanen IL-7 (R&D Systems) und 10 ng/ml rekombinantem humanen IL-15 (R&D Systems) sowie mit bzw. ohne blockierende Anti-CD1d-Antikörper (CD1d51, 10microg/ml). Die absolute Zahl an NKT-Zellen und der Expansionsfaktor wurden mittels FACS-Analysen bestimmt.

### Ergebnisse

### Differenzierung der MUTZ-3 Zellen zu effektiven DC unterschiedlicher Differenzierungsstadin und Effektorstadien

### MUTZ-3-zellen erwerben nach Zytokingabe den Phänotyp unreifer DC-

Als erstes bestimmten wir das Potential leukämischer Zelllinien zu differenzieren in Anwesenheit von Zytokinen, die routinemäßig für die Induktion von DC Zellen verwendet werden. Insbesondere untersuchten wir die mit Zytokinen behandelten Zelllinien, ob die Expression von CD1a, einem Hauptcharakteristikum für unreife dendritische Zellen (iDC), auf der Oberfläche der Zellen induziert wurde. Drei der sechs getesteten Zelllinien (MUTZ-3, KG-1, THP-1) reagierten auf den Zytokin-Cocktail GM-CSF, IL-4 und niedrigdosiertes TNFα. Der Anteil CD1a-positiver Zellen nach 7 Tagen in Kultur war am höchsten bei der Zelllinie MUTZ-3 (20%), während die Zelllinien KG-1 und THP-1 10% bzw. 5% CDIa-positive Zellen aufwiesen (Tabelle 1). Bei diesen letzten zwei Zelllinien ging die Differenzierung mit einer deutlichen Expression des DC-Reifungsmarkers CD83 einher, was frühere Ergebnisse bestätigt (Hulette et al. 2001, Arch Dermatol Res 293(3):147-158; St Louis et al. 1999, J Immunol 162(6):3237-3248). KG-1 und THP-1 reagierten nicht auf weitere Zytokin-Stimuli, auch konnte keine weitere Änderung des CD1a/CD83-Phänotyps beobachtet werden. Auf den übrigen 3 untersuchten Zelllinien wurden weder CD1a noch CD83 detektiert. Alle getesteten Zelllinien exprimierten den GM-CSF-Rezeptor (CD116), aber nur die Zelllinie MUTZ-3 auch den IL-4-Rezeptor (CD124). Das zeigt die einzigartige Fähigkeit von MUTZ-3 Zellen, CD1a positiv zu werden ohne gleichzeitig auch CD83 zu exprimieren, d.h. den iDC Phänotyp zu erwerben.

### MUTZ-3 ist ein CD34-positives DC-Differenzierungsmodell, das von Vorläuferzellen abgeleitet ist

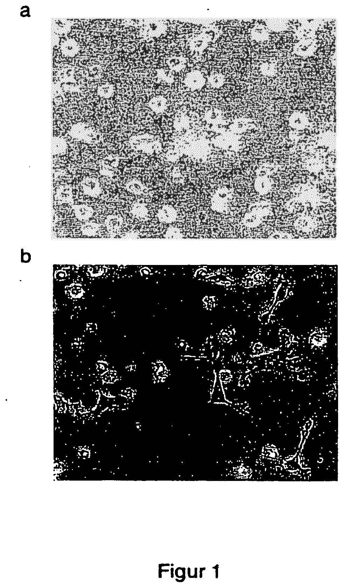

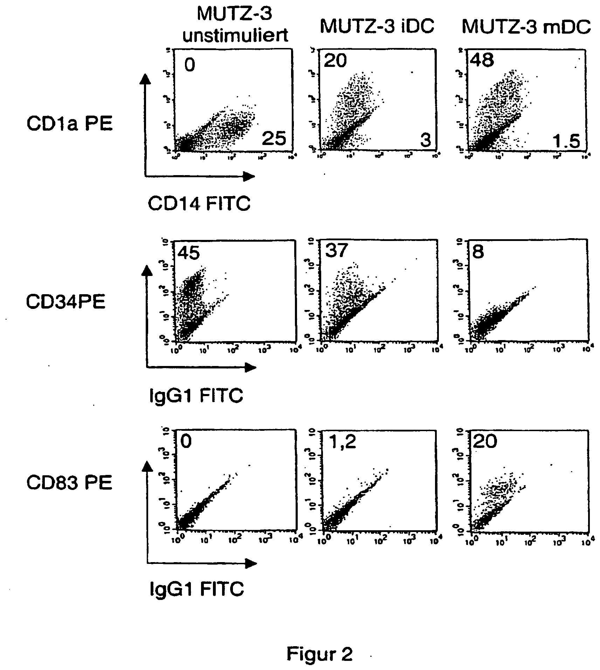

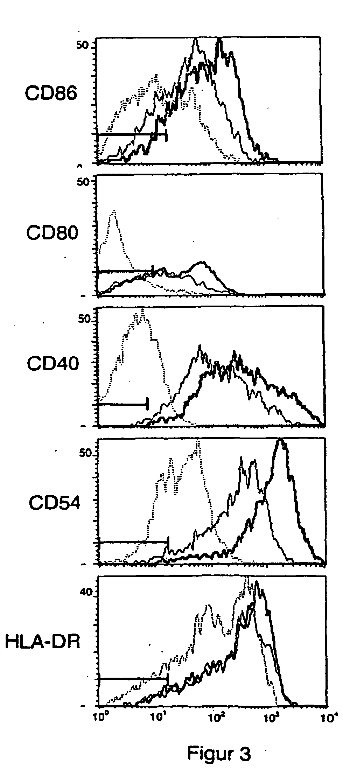

Neben der Neo-Expression von CD1a wurden weitere morphologische und phänotypische Veränderungen nach der Zytokin-Stimulation von MUTZ-3 Zellen beobachtet. MUTZ-3 Zellen waren typischerweise nicht-adhärente, runde oder ein wenig gelappte Zellen. Nach der Differenzierung waren die MUTZ-3 iDC Zellen nur noch locker adhärent, bildeten Klumpen aus großen Zellen und entwickelten haarähnliche, zytoplasmatische Projektionen, ein morphologisches Charakteristikum von DC Zellen (Fig. 1a, b).
Die Analyse der Zelloberflächen-Marker zeigte, daß unstimulierte MUTZ-3-Zellen CD14, CD86, CD54 und CD40 schwach exprimieren und CD34 und HLA-DR stark (Fig. 2 und 3). Nach Induktion der CD1a Expression auf der ZellOberfläche wurde eine herunter-regulierte CD14-(Monozytenmarker) und CD34-Expression. (Marker hämatopoetischer Vorläuferzellen) beobachtet. Die Expression der co-stimulatorischen und Adhäsions-Moleküle CD80, CD86, CD40, CD54 und HLA-DR war stark heraufreguliert auf den MUTZ-3 iDC Zellen im Vergleich zur unstimulierten Zellpopulation (Fig.3) . Die Stimulation der MUTZ--3 iDC Zellen mit TNFα induzierte die Expression des DC-Reifungsmarkers CD83 mit einer weiteren Steigerung der CD1a Expression von und allen co-stimulatorischen Molekülen. Ähnliche Beobachtungen wurden gemacht, wenn die MUTZ-3 iDC Zellen mit LPS, CD40-Ligand-transfizierten J558 Zellen oder PolylC zur Reifung gebracht wurden (Ergebnisse werden nicht gezeigt). Keine weitere Proliferation wurde beobachtet, wenn zu den MUTZ--3 iDC oder reifen DC (mDC) Zellen Zytokine zugegeben wurden.
MUTZ-3 Zellen können demzufolge unter dem Einfluß von GM-CSF, IL-4 und niedrigdosiertem TNFα in DC Zellen differenzieren (MUTZ-3-DC), wobei sie zwei verschiedene Differenzierungsstadien durchlaufen - einen unreifen (MUTZ-3 iDC) und einen reifen Phänotyp (MUTZ-3 mDC).

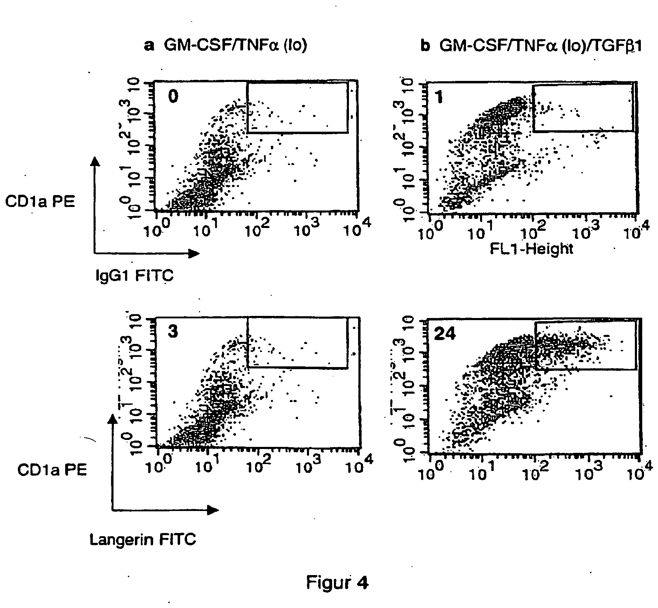

Die Herab-Regulation von CD34 und CD14 deutet daraufhin, dass MUTZ-3 Zellen eine Population von Vorläuferzellen bei der Differenzierung von CD34-positiven Stammzellen darstellt. Bei der Differenzierung von CD34-positiven Stammzellen entstehen wenigstens zwei Vorläuferzelltypen, welche ultimativ zu interstitialen und Langerhans' Zellen (LC) reifen. Um zu bestimmen, ob sich MUTZ-3 Zellen zu LC ähnlichen Zellen entwickeln können, haben wir MUTZ-3 Zellen in An- oder Abwesenheit von TGFbeta1 kulti-viert. TGFbeta1 ist bekannt dafür bei DC Zellen, die von CD34-positiven Zellen abstammen, einen LC-Phänotyp zu induzieren (Caux et al. 1997, Blood 90 (4): 1458-1470). Wir beobachteten, dass unter dem Einfluß von TGFbeta1 nicht nur der Anteil an CD1a positiven MUTZ-3 Zellen von 20% auf 80% anstieg, sondern auch eine starke Langerin / CD1a Doppelfärbung auftrat (Fig. 4). Letzteres zeigt, dass diese Zellen bestimmte Charakteristika von LC Zellen aufweisen.

### MUTZ-3 DC Zellen induzieren die Proliferation von allogenen Lymphozyten

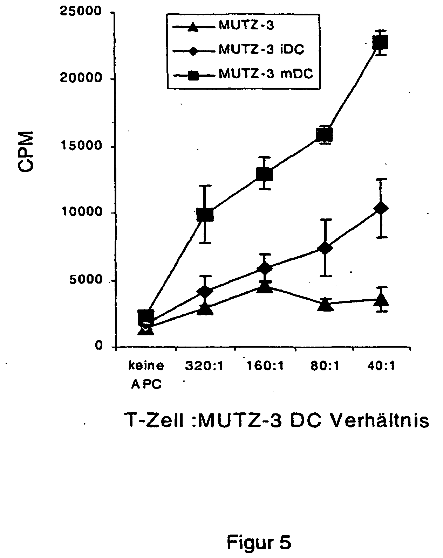

MUTZ-3 mDC zellen waren in der Lage in gemischten Lymphozyten-Reaktionen die Proliferation von allogenen T-Zellen zu stimulieren, und zwar zu einem höheren Grad als MUTZ-3 iDC oder unstimulierte MUTZ-3. Zellen. Es wurde ein 6-10 fach höherer [³H] -Thymidin Einbau (Lymphozyten Proliferation) verglichen mit unstimulierten MUTZ-3 Zellen und ein 2-3 fach höherer [³H]-Thymidin Einbau verglichen mit MUTZ-3 iDC Zellen gemessen bei einem MUTZ-3/PBL Verhältnis von 40:1 (Fig. 5). Wahrscheinlich spiegelt diese erhöhte stimulatorische Eigenschaft von MUTZ-3 mDC verglichen mit MUTZ-3 iDC Zellen den beobachteten Anstieg der Expression der co-stimulatorischen und Adhäsions-Marker CD80, CD86, CD40 und CD54 wider (wie in Fig.3 gezeigt).

### MUTZ-3 DC Zellen reagieren auf Th-polarisierende Stimuli und bekommen einen DC1 oder DC2 Phänotyp während der Reifung

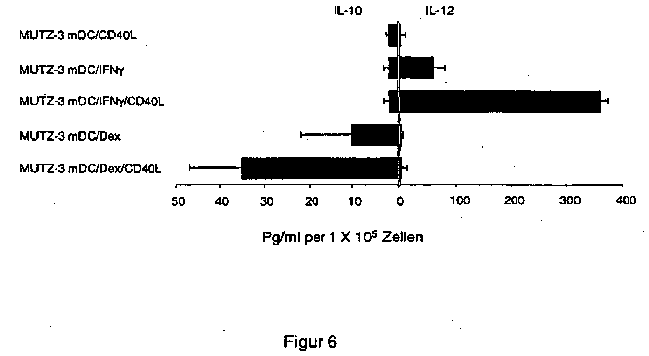

DC Zellen können IL-12 sekretieren, einem potenten Typ 1 T-Zellen induzierenden Zytokine (Kalinski et al 1998, J Immunol 161 (6): 2804-2809). Es konnte außerdem gezeigt werden, dass unter dem Einfluß gewisser Stimuli nicht-vorprogrammierte iDC Zellen die Fähigkeit erhalten, hauptsächlich IL-12 (DC1 Phänotyp) oder das Typ 2 induzierende Zytokine IL-10 (DC2 Phänotyp) zu sekretieren (Vieira et al. 2000, J Immunol 164 (9): 4507-4512; Langenkamp et al. 2000, Nat Immunol 1 (4): 311-316). Um zu untersuchen, ob MUTZ-3 iDC Zellen den DC1 oder DC2 Phänotyp entwickeln können, wurde die Reifung der MUTZ-3 iDC Zellen in Anwesenheit von IFN-gamma oder Dexamethason induziert. Wenn MUTZ-3 mDC Zellen (nach Reifung in Anwesenheit von TNFalpha) mit oder ohne CD40 Liganden transfizierten J558 Zellen stimuliert wurden, wurden geringe Mengen an IL-10 und IL-12 produziert (Fig. 6). Demgegenüber ergab die Reifung von MUTZ-3 iDC Zellen in Anwesenheit von IFN-gamma eine IL-12 Produktion, die darüber hinaus kräftig anstieg, wenn die Zellen durch die transfizierten J558 Zellen weiter stimuliert wurden (Post-Maturation). Ganz im Gegensatz dazu wurde überhaupt kein IL-12 von MUTZ-3 mDC Zellen produziert, wenn diese in Anwesenheit von Dexamethason zur Reifung gelangten. In diesen Zellkulturen konnte aber eine erhöhte IL-10 Produktion nachgewiesen werden. Diese Ergebnisse zeigen, dass unter geeigneten Bedingungen nicht-vorprogrammierte MUTZ-3 DC Zellen zum DC1 oder DC2 Phänotyp verändert werden können.

### MUTZ-3 Zellen als effektive DC, die die Fähigkeit besitzten Antigene zu prozessieren und zu präsentieren und eine Immunantwort zu induzieren

Eine zentrale Funktion von DC Zellen als professionelle Antigen präsentierende Zellen (APC) ist ihre Fähigkeit, CD4- und CD8-positive T-Zellen zu stimulieren, sowie (wie erst kürzlich gezeigt) Lipide und hydrophobe Antigene NKT Zellen zu präsentieren. Deshalb haben wir untersucht, ob auch MUTZ-3 DC Zellen in der Lage sind auf diesen Wegen spezifisch Antigene zu prozessieren und zu präsentieren.

### MUTZ-3 DC Zellen aktivieren Influenza-spezifische, zytotoxische T-Lymphozyten über MHC Klasse I

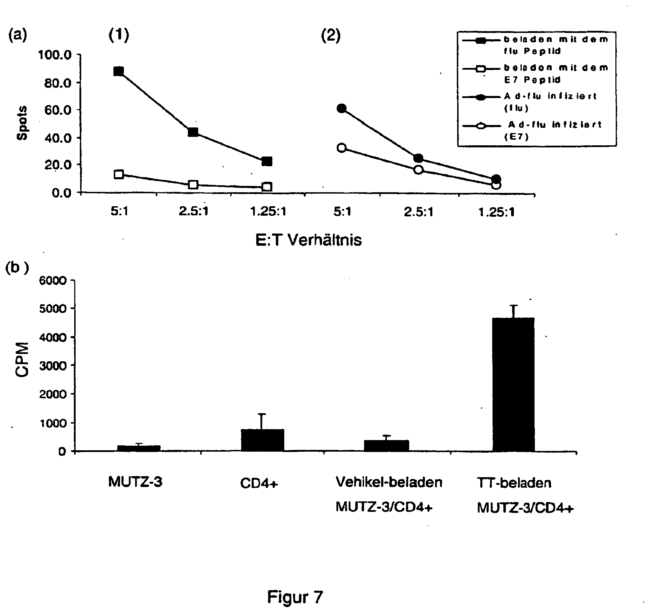

Die molekulare Typisierung ergab, daß MUTZ-3 Zellen positiv waren für die HLA Antigene HLA-A2, HLA-A3, HLA-B44, HLA-DR10, HLA-DR11, HLA-DR52, HLA-DQ5 und HLA-DQ7. Die HLA-A2 Expression wurde bestätigt durch eine FACS-Analyse unter Verwendung der monoklonalen Antikörper MA2.1 und BB 7.1 (Ergebnisse nicht gezeigt). Wir haben nun untersucht, ob MUTZ-3 DC Zellen fähig sind, Antigene über das HLA-A2 Klasse I Molekül zu prozessieren und zu präsentieren. MUTZ-3 DC Zellen wurden beladen mit dem immunodominanten ,A2 bindenden M1 Häminfluenza (flu) Peptid oder die Zellen wurden mit Adenoviren infiziert, die für die gesamte M1 Sequenz kodieren (um die Fähigkeit zur HLA Klasse I Prozessierung zu testen). In beiden Fällen wurden T2 Zellen, die mit dem M1 flu Peptid oder als Kontrolle mit dem vom HPV-abstammenden E7 Peptid beladen wurden, als Stimulator-Zellen im IFNgamma-ELISPOT Assay für zytotoxische T-Lymphozyten (CTL) benutzt, welche bei der Co-Kultivierung von MUTZ-3 DC und T-Zellen entstanden sein könnten. Unstimulierte T-Zellen wurden hinzugefügt, um die Basislinie der flu-spezifischen CTL Reaktion zu bestimmen. Keine spezifische CTL Antwort wurde unter diesen Bedingungen beobachtet (Ergebnisse nicht gezeigt). Eine HLA-A2 beschränkte, flu-spezifische CTL Expansion konnte detektiert werden nach Co-Kultivierung der CTL mit MUTZ-3 DC Zellen, die entweder mit dem flu Peptid beladen waren oder mit den M1 kodierenden Adenoviren infiziert worden waren (Fig. 7a 1, 2). Diese Ergebnisse zeigen, daß MUTZ-3 DC Zellen flu Peptide prozessieren und präsentieren können, was zu einer Stimulation von flu spezifischen, MHC Klasse I-beschränkten CTL führt.

### MUTZ-3 DC Zellen induzieren MART-1-spezifische, zytotoxische T-Lymphozyten über MHC-Klasse I

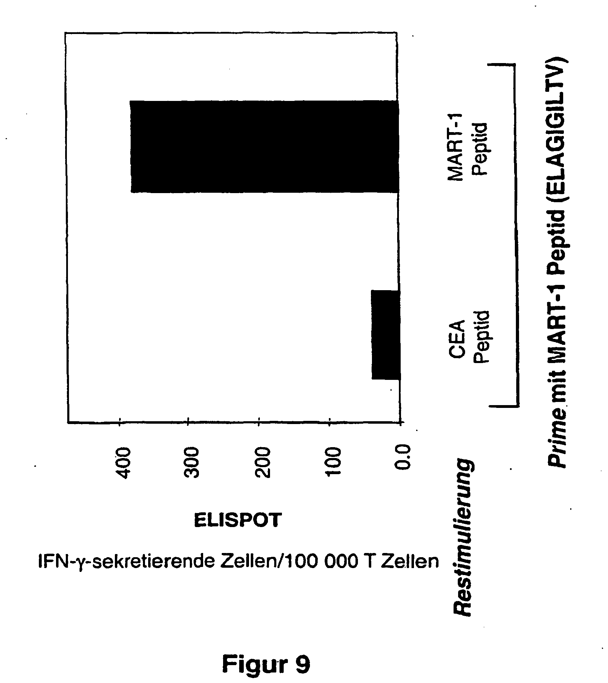

Eine HLA-A2-abhängige, MART-1-spezifische CTL-Expansion und - Aktivierung (IFNgamma-Sekretion) konnte detektiert werden nach Ko-kultivierung der CTL mit MUTZ-3-DC, die mit dem modifizierten MART-1 Peptid ELAGIGILTV beladen wurden (Fig. 9). Diese Ergebnisse zeigen, daß MUTZ-3-DC über MHC Klasse I naive CTL sensibilisieren können.

### Tumorzelllysat-beladene MUTZ-3-DC induzieren zytotoxische T-Lymphozyten die für verschiedene Tumorantigene spezifisch sind

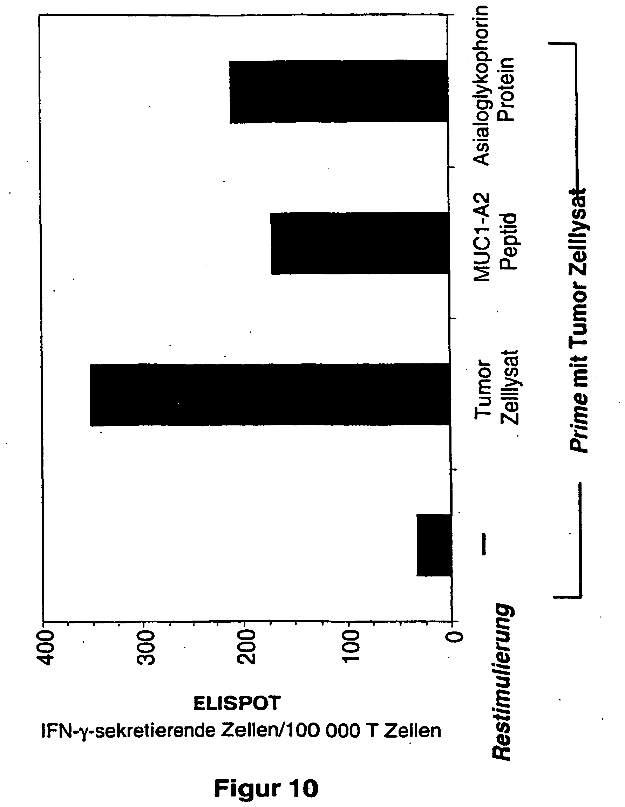

HLA-A2-abhängige, Tumorzelllysat-spezifische CTL-Expansion und - Aktivierung (IFNγ-Sekretion) konnte detektiert werden nach Ko-Kultivierung der CTL mit MUTZ-3-DC, die mit Tumorzelllysat beladen wurden (Fig 10). Diese CTL konnten auch durch Restimulierung mit MUC1-Peptid LLLLTVLTV, und durch Restimulierung mit dem Protein Asialoglykophorin aktiviert werden. Diese Ergebnisse zeigen, daß MUTZ-3-DC eine polyspezifische zelluläre Anti-Tumor-Immunantwort induzieren können.

### Generierung von unreifen Mutz-3 (iDC) aus Vorläuferzellen mit GM-CSF, TNF-alpha und verschiedenen IL-4 Konzentration oder IL-13

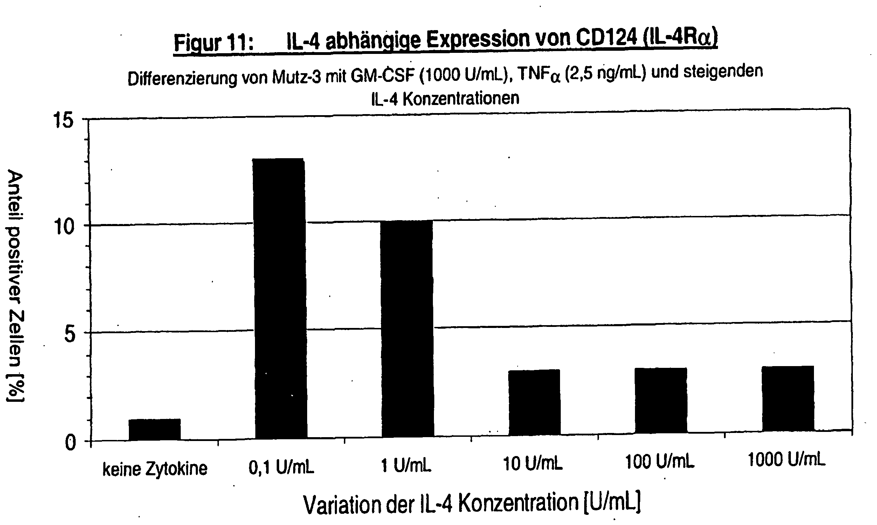

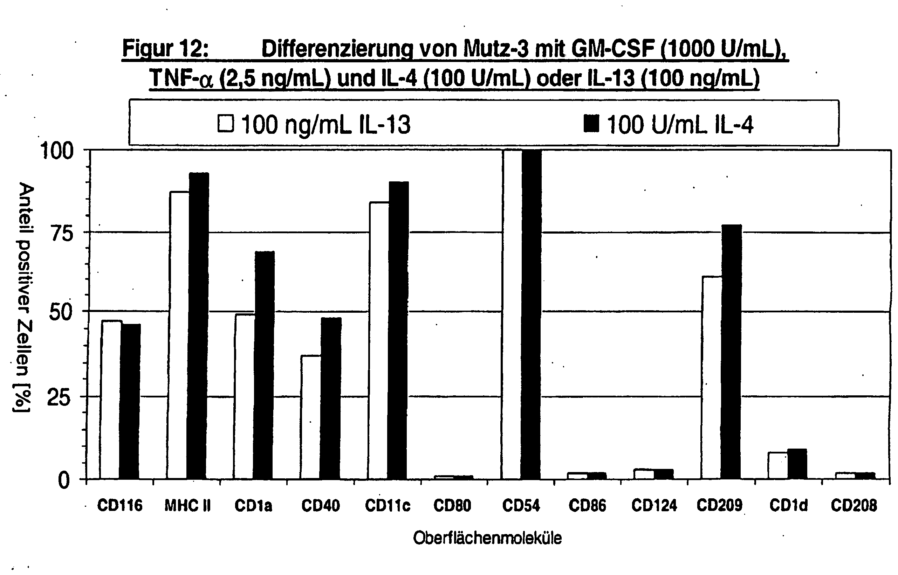

Mutz-3 Zellen aus der laufenden Kultur wurden 2 mal mit PBS gewaschen und mit einer Zelldichte von 1 x 10⁵ Zellen/mL in einem Volumen von 5 mL Kulturmedium in eine 6-Lochplatte eingesät und für 7 Tage mit GM-CSF (1000 U/mL, Leukomax /Novartis), und niedrigdosiertem TNFalpha (2,5 ng/mL, Peprotech) und verschiedenen IL-4 Konzentrationen (zwischen 0,1 U/mL und 1000 U/mL, Peprotech) inkubiert. In einem weiteren Versuch wurde IL-4 durch IL-13 (100 ng/mL) ersetzt. Diese Konzentration entspricht etwa der 40-fachen Konzentration verwendeten IL-4 Konzentration (100U/ml). Jeden zweiten bis dritten Tag erfolgte ein Zytokinzufütterung. Nach 7 Tagen Inkubation erfolgte eine durchflusszytometrische Charakterisierung der Zellen.(siehe Fig. 11 und 12)

### Stimulation von TT spezifischen, CD4-positiven T-Zellen durch TT-gepulste MUTZ-3 iDC Zellen

Die Fähigkeit der Prozessierung von Peptiden über den MHC Klasse II Weg, wurde untersucht, indem MUTZ-3 iDC Zellen mit Peptiden gepulst beladen wurden, die von dem "common recall" TT Antigen stammten, und anschließend mit allogenen CD4-positiven, teilweise im HLA-Typ übereinstimmenden T-Zellen co-kultiviert wurden. Es wurde eine starke Stimulation der TT spezifischen CD4-positive T-Zellen beobachtet, wenn MUTZ-3 iDC Zellen mit TT Peptiden im Vergleich zum Vehicle als Kontrolle gepulst beladen wurden, wobei die Kontrollwerte ähnlich niedrig waren wie für CD4-positive Zellen alleine (Fig. 7b). Diese Ergebnisse zeigen, dass MUTZ-3 Zellen in der Lage sind, Antigene über den MHC Klasse II Weg zu prozessieren und zu präsentieren.

### Glykolipid Präsentation durch MUTZ-3 DC Zellen für Valpha24-positive / Vbetall-positive NKT Zellen

CD1 Moleküle repräsentieren eine spezialisierte Klasse von Antigen präsentierenden Molekülen, welche in der Lage sind, Lipide, Glykolipide und hydrophobe Peptide zu präsentieren. Es konnte gezeigt werden, dass das Glykolipid α-GalCer über CD1d Vα24-positiven / Vβ11-positiven NKT Zellen präsentiert werden kann (Brossay et al. 1998, J. Exp. Med. 188 (8): 1521-1528). Um zu untersuchen, ob MUTZ-3 DC Zellen in der Lage sind, α-GalCer zu präsentieren, haben wir zunächst gezeigt, daß MUTZ-3 DC Zellen das CD1d Molekül exprimieren (Ergebnisse nicht gezeigt). MUTZ-3 iDC und mDC Zellen wurden dann beladen mit dem α-GalCer oder Vehicle und co-kultiviert mit gereinigten NKT Zellen für 7 Tage in Anwesenheit von 10 ng/ml IL-7 und IL-15 (van der Vliet et al. 2001, J. Immunol. Methode 247 (1-2): 61-72). α-GalCer beladene MUTZ-3 mDC Zellen konnten besser NKT Zellen induzieren als MUTZ-3 iDC Zellen (sowohl α-GalCer als auch vehicle-beladen) und Vehicle beladene MUTZ-3 mDC Zellen. Die Aufhebung der Antigen Präsentation durch Blockierung von CD1d bestätigte die Schlußfolgerung, daß MUTZ-3 mDC Zellen in der Lage sind, Glykolipid Antigene über die nichtklassischen Antigen präsentierenden CD1d Moleküle zu präsentieren (Fig.. 8).

### Legenden

**Tabelle 1. FACS Analyse der CD1a und CD83 Expression in Leukämia Zelllinien.** Die CD1a und CD83 Expression wurde in der Durchflußzytometrie untersucht nach 7 Tagen Inkubation mit den Zytokinen. Bei MUTZ-3 Zellen ist eine Neo-Expression von CD1a aber nicht von CD83 zu beobachten. Eine geringere Induktion der CD1a Expression mit assoziierter CD83 Expression wurde für KG-1 und in einem noch geringeren Maße auch für THP-1 Zellen gemessen. ^{a} %Positive Zellen repräsentiert die gesamte Zahl an Zellen, die für einen bestimmten CD-Marker positiv gefärbt waren, innerhalb einer abgegrenzten ("gated") Zellpopulation. ^{b} Zellen, die mit PE-konjugierten anti CD1a und FITC-konjugierten anti CD83 monoklonalen Antikörpern gefärbt wurden, repräsentieren Doppel-positive Zellen. ^{c} Zellen wurden mit FITCkonjugiertem anti CD116 monoklonalen Antikörpern gefärbt. ^{d} publiziert in Drexler, H.G. 2001, The Leukemia-Lymphoma Cell Line Facts Book, Academic Press.
**Figur 1****. Mikroskopische Aufnahmen von differenzierten MUTZ-3 Zellen nach Zugabe von Zytokinen.** a) Unstimulierte MUTZ-3 Zellen, b) MUTZ-3 iDC nach 7 Tagen Kultur in Anwesenheit von GM-CSF, IL-4 und geringen Konzentrationen von TNFalpha. Die Zellen sind nur noch locker adhärent und zeigen eine dendritische Morphologie (40 fache Vergrößerung).
**Figur 2****. MUTZ-3 DC zeigen Charakteristika für unreife und reife DC in Anwesenheit von Zytokinen.** Die Scatter Plot Darstellung illustriert den Phänotyp a) der unstimulierten MUTZ-3 Zellen, b) der unreifen MUTZ-3 iDC und c) der TNFalpha induzierten reifen MUTZ-3 mDC. Die Zahlen beziehen sich auf den Prozentsatz der Zellen, die positiv sind für den jeweiligen CD Marker. Alle Zellen wurden mit PE- oder FITC-konjugierten, Antigen spezifischen, monoklonalen Antikörpern gefärbt. Die Daten stammen von einem Experiment, das representativ für fünf Experimente ist.
**Figur 3****. Die Differenzierung der MUTZ-3 Zellen ist assoziiert mit der Induktion der Expression von costimulatorischen Molekülen.** Die FACS Analyse ergibt eine Induktion der co-stimulatorischen Moleküle CD86 und CD40, des Adhäsions-Moleküls CD54 und des HLA Klasse II Moleküls HLA-DR während der MUTZ-3 Differenzierung, unstimulierte MUTZ-3 (gepunktete Linie), unreife MUTZ-3 iDC (durchgezogene Linie) und reife MUTZ-3 mDC (durchgezogenen dicke Linie). Die Daten stammen von einem Experiment, das representativ für fünf Experimente ist.
**Figur 4****. TGFbebtal induziert die Expression des LC-assoziierten oberflächenmoleküls Langerin auf MUTZ-3 zellen.** CD34-positive MUTZ-3 Zellen wurden zunächst in Anwesenheit von GM-CSF/TNFalpha und anschließend in Anoder Abwesenheit von TGFbetal kultiviert. Die Zahlen in der oberen linken Ecke beziehen sich auf den Prozentsatz an CD1a/Langerin doppel-positiven Zellen innerhalb einer abgegrenzten ("gated") Zellpopulation bzw den Prozentsatz an Zellen, die zur Kontrolle mit einem isotypischen Antikörper gefärbt wurden. Die Daten stammen von einem Experiment, das representativ für drei Experimente ist.
**Figur 5****. Die Fähigkeit von MUTZ-3 Zellen, Lymphozyten zu stimulieren.** Unstimulierte MUTZ-3, unreife MUTZ-3 iDC und reife MUTZ-3 mDC wurden mit Lymphozyten, die im MHC nicht übereinstimmten, co-kultiviert in einer allogenen gemischten Lymphozyten Reaktion. MUTZ-3 mDC wiesen eine starke stimulatorische Kapazität auf verglichen mit unstimulierten MUTZ-3 Zellen (6,3 fache Differenz im [3H] Thymidin Einbau verglichen mit unstimulierten Zellen) und eine 2, 3 fache Differenz verglichen mit MUTZ-3 iDC. Die Daten stammen von einem Experiment, das representativ für vier Experimente ist.
**Figur 6****. Nicht-vorprogrammierte MUTZ-3 iDC Zellen können zum DC1 oder DC2 Phänotyp verändert werden während der Reifung unter Einfluß von IFNgamma oder Dexamethason.** MUTZ-3 iDC, die in Anwesenheit von IFNgamma kultiviert wurden, sekretieren IL-12. Keine IL-12 Produktion kann beobachtet werden, wenn die Zellen mit Dexamethason kultiviert wurden. In ähnlcher Weise sekretieren die Zellen kein IL-10, wenn sie mit IFNgamma behandelt wurden. IL-12 und IL-10 Konzentrationen wurden im ELISA bestimmt. Die Zytokine Konzentrationen sind in pg/ml pro 10⁵ Zellen dargestellt. Die Daten sind representativ für vier indivuelle Experimente.
**Figur 7****. MUTZ-3-Zellen besitzen die Fähigkeit der Antigen-Prozessierung und Präsentation** (a) MHC Klasse I Präsentation, MUTZ-3 iDC stimulieren eine flu-spezifische CTL Reaktion durch Präsentation des auf das HLA-A2.1 beschränkten flu Peptids. (1) MUTZ-3-DC wurden mit dem HLA-A2.1 bindenden, vom Häminfuenza stammenden Matrixprotein M1₅₈₋₆₆ beladen und co-kultiviert mit CD8-positiven T-Zellen. Zum Nachweis der CTL Proliferation wurde die Produktion von IFNγ durch CTLs gemessen, die mit T2-Zellen als Ziel-Zellen co-kultiviert wurden. Die T2-Zellen waren entweder mit dem M1 flu Peptid (schwarze Quadrate) oder als Kontrolle mit dem vom HPV16 abstammenden Pepid E7 (weiße Quadrate) beladen. (2)MUTZ-3-DC wurden mit rekombinanten Adenoviren infiziert, die das M1 Matrixprotein Gen enthielten und anschließend co-kultiviert wie oben beschrieben. CTLS wurden dann erneut mit T2-Zellen stimuliert, die entweder mit dem M1 flu Peptid (schwarze Kreise) oder mit E7 Peptid (weiße Kreise) beladen waren. Die Daten stammen von einem Experiment, das repräsentativ für drei Experimente ist. (b) MHC Klasse II Antigen Präsentation, MUTZ-3 mDC prozessieren und präsentieren Peptide, die von dem "common recall" Antigen TT stammen, und stimulieren TT spezifische CD4-positive T-Zellen. Die Daten stammen von einem Experiment, das repräsentativ für drei Experimente ist.

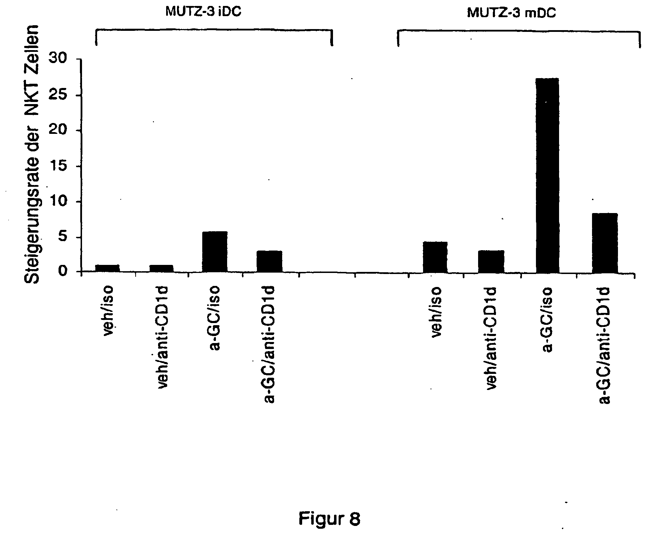

Figur 8 . Präsentation von α-GalCer über CD1d. MUTZ-3 iDC wurden mit dem α-GalCer oder als Kontrolle mit Vehikel (DMSO) beladen und anschließend für 48h in An- oder Abwesenheit von höher dosiertem TNFα kultiviert. mDC wurden dann für 9 Tage in Anwesenheit von IL-7 und IL-15 mit oder ohne CD1d blockierenden Antikörper co-kultiviert mit NKT-Zellen, die von gesunden Spendern isoliert worden waren. Die Ergebnisse zeigen die relative Ausbeute von NKT-Zellen nach Co-Kultivierung mit MUTZ-3 iDC und mDC, welche vorher mit Vehikel und α-GalCer beladen wurden, mit oder ohne Blockierung der α-GalCer-Präsentation durch den CD1d-blockierenden Antikörper. Die Daten stammen von einem Experiment, das repräsentativ für drei Experiment ist.
**Figur 9** **MUTZ-3-DC können naive CTL sensibilisieren.**
   CTL wurden mit MART-1 ELAGIGILTV Peptid-beladene MUTZ-3-DC stimuliert (Prime) und nach eine Woche über Nacht mit MART-1 ELAGIGILTV oder CEA IMIGVLVGV Peptid-beladene T2-Zellen restimuliert. Das IFNgamma ELISPOT zeigte eine starke antigenspezifische (MART-1) Aktivierung der CTL, durch Restimulierung mit einem irrelevanten Antigen (CEA)ergab nur eine sehr schwache Aktivierung der Zellen.
**Figur 10** **MUTZ-3-DC können eine polyspezifische Anti-Tumor-CTL Antwort induzieren.**
   CTL wurden mit Tumorzelllysat-beladene MUTZ-3-DC stimuliert (Prime) und nach eine Woche mit Tumorzelllysat-beladene MUTZ-3-DC, mit Asialoglykophorin-beladene MUTZ-3-DC, oder mit MUC1 LLLLTVLTV Peptid-beladene T2-Zellen restimuliert. Das IFNgamma ELISPOT zeigte starke Aktivierung der CTL durch Restimulierung der Zellen mit Tumorzelllysaten, mit dem MUC-1-Peptid und mit dem Asialoglykophorin.
**Figur 11****:** Mutz-3 Zellen aus der laufenden Kultur wurden für 7 Tage mit GM-CSF (1000 U/mL), niedrigdosiertem TNF-alpha (2,5 ng/mL) und verschiedenen IL-4 Konzentrationen (zwischen 0,1 U/mL und 1000 U/mL) inkubiert. Die Ergebnisse zeigen, dass mit steigender IL-4 Konzentration eine Reduktion der CD124 Expression beobachtet werden kann.
**Figur 12****:** Mutz-3 Zellen aus der laufenden Kultur wurden für 7 Tage mit GM-CSF (1000 U/mL), niedrigdosiertem TNF-alpha (2,5 ng/mL) und vergleichend mit IL-4 (100 U/mL) oder IL13 (100 ng/mL) inkubiert. Die Konzentration des IL-13 entspricht etwa der 40-fachen Konzentration des verwendeten IL-4. Die durchflusszytometrische Charakterisierung verschiedener Oberflächenmoleküle zeigt, dass durch eine 7-tägige Inkubation mit IL-13 anstelle von IL-4, neben GM-CSF und niedrigdosiertem TNF-alpha, eine vergleichbare Expression der Oberflächenmoleküle beobachtet werden kann.
Im Kontext der Erfindung bedeutet der Ausdruck "sensibilisieren", T-Lymphozyten in einen Status versetzen, in dem sie für einen antigenspezifische Stimulus empfänglich sind.

**Tabelle 1: FACS-Analyse der CD1a- und CD83-Expression auf leukämischen Zelllinien. Die CD1a- und CD83-Expression wurde mittels Durchflusszytometrie am 7.Tag nach Zytokingabe untersucht. Die CD1a, jedoch nicht die CD83 Expression kann in MUTZ-3 Zellen induziert werden. KG-1, und in einem geringen Ausmaß TH-1, erlangen CD1a(gering) Expression in Verbindung mit CD83 Expression.**

| Zellinie | % Positve Zellen^{a} | | Zytokin Rezeptor Expression | |
|---|---|---|---|---|
| | CD1a^{b} | CD83^{b} | CD116 | CD124 |
| | | | (GM-CSF Rezeptor)^{c} | (IL-4 Rezeptor)^{d} |
| MUTZ-3 | 38 | 0 | + | + |
| KG-1 | 10 | 10 | + | - |
| THP-1 | 5 | 5 | + | - |
| HL-60 | 0 | 0 | + | - |
| U937 | 0 | 0 | + | - |
| K562 | 0 | 0 | + | - |
| ^{a} % positive Zellen repräsentiert die Gesamtzellzahl positive gefärbter Zellen für einen bestimmten DC-Marker innerhalb einer eingegrenzten Zellpopul ation. | | | | |
| ^{b} Zellen wurden mit PE-markierten anti-CD1a und FITC-markierten anti-CD83 Monoklonalen Antikörpern gefärbt, dieses Ergebnis repräsentiert dopppelt positive Zellen. | | | | |
| ^{c} gefärbt mit anti-CD16-FITC-markierten Monoklonalen Antikörpern. | | | | |
| ^{d} von Drexler, H.G., 2001. The Leukemia-Lymphoma Cell Line Facts Book. Academic Press. | | | | |

## Patentansprüche

1. Verfahren zur Herstellung von effektiven dendritischen Zellen oder Zelllinien, **dadurch gekennzeichnet, dass** die CD124 und CD116 positive MUTZ-3 Zelllinie mit stimulatorischen Molekülen, zeit gleich oder sequentiell zeit versetzt, in Kontakt gebracht und die effektiven dendritischen Zellen oder Zelllinien erhalten werden, wobei als die stimulatorischen Moleküle GM-CSF, TNF-alpha, LPS, PGE2, CD40 Ligand, poly inosinische-poly cytidylische Säure (polylC), PMA, TGFbeta1, IL-7 oder IL-4 verwendet werden.

2. Verfahren nach Anspruch 1, **dadurch gekennzeichnet, dass** als effektive dendritische Zellen oder Zelllinien, Zellen oder Zelllinien hergestellt werden, die einen Phänotyp von interstitiellen dendritischen Zellen, Langerhans' dendritischen Zellen, CD83 positiven Zellen, unreifen dendritischen Zellen und/oder reifen dendritischen Zellen haben.

3. Verfahren nach einem der vorhergehenden Ansprüche, **dadurch gekennzeichnet, dass** die CD124 und CD116 positive Zelllinie oder Zellen CD34 positiv ist/sind.

4. Verfahren nach einem der vorhergehenden Ansprüche, **dadurch gekennzeichnet, dass** in die CD116 und CD124 positive Zelllinie oder Zellen das Gen für CD34 eingebracht wird.

5. Verfahren nach einem der vorhergehenden Ansprüche, **dadurch gekennzeichnet, dass** in die Zelllinie oder Zellen weitere Gene eingebracht werden, die beispielsweise Rezeptoren oder Inhibitoren für stimulatorische Moleküle codieren und/oder exprimieren.

6. Verfahren nach einem der vorhergehenden Ansprüche, **dadurch gekennzeichnet, dass** in die Zelllinie oder Zellen mindestens ein Immuntherapeutikagen eingebracht wird, wobei das Immuntherapeutikagen ein Gen ist, das ein Tumorantigen, virales Antigen, Antigen von Parasiten, Bakterien oder Mikroorganismen kodiert.

7. Verfahren nach einem der vorhergehenden Ansprüche, **dadurch gekennzeichnet, dass** die Zelllinie oder Zellen mit anderen Zellen oder Zelllinien fusioniert wird/werden.

8. Verfahren nach einem der vorhergehenden Ansprüche, **dadurch gekennzeichnet, dass** durch die unterschiedlichen stimulatorischen Moleküle, ihre Dosierung und/oder durch die Reihenfolge des in Kontaktbringens effektive dendritische Zelllinien oder Zellen unterschiedlicher Aktivierungs- und/oder Effektorstadien gewonnen werden.

9. Verfahren nach einem der vorhergehenden Ansprüche, **dadurch gekennzeichnet, dass** die CD124 und CD116 positive Zelllinie und/oder die effektiven dendritischen Zellen oder Zelllinien in die Apoptose oder Nekrose gebracht wird/werden, beispielsweise durch Bestrahlung der Zellen.

10. Verfahren nach einem der vorhergehenden Ansprüche, **dadurch gekennzeichnet, dass** in die CD124 und CD116 positiven Zelllinie oder Zellen und/oder effektiven dendritischen Zellen oder Zelllinien mindestens ein Suizid-Gen eingebracht wird.

11. Verfahren nach einem der vorhergehenden Ansprüche, **dadurch gekennzeichnet, dass** durch die unterschiedlichen stimulatorischen Moleküle, ihre Dosierung und/oder Reihenfolge des in Kontaktbringens effektive dendritische Zelllinien oder Zellen hergestellt werden, die vom DC Typ 1 oder DC Typ 2 sind.

12. Verfahren nach einem der vorhergehenden Ansprüche, **dadurch gekennzeichnet, dass** die effektiven dendritischen Zelllinien oder Zellen mit den stimulatorischen Molekülen in Kontakt gebracht und effektive reife dendritische Zelllinien oder Zellen erhalten werden.

13. Effektive dendritische menschliche MUTZ-3 Zellen oder MUTZ-3 Zelllinie, erhältlich nach einem Verfahren der Ansprüche 1 bis 12.

14. Effektive dendritische menschliche MUTZ-3 Zellen oder MUTZ-3 Zelllinie nach Anspruch 13, die mit mindestens einem Antigen beladen sind/ist.

15. Effektive dendritische menschliche MUTZ-3 Zellen oder MUTZ-3 Zelllinie nach Anspruch 14, wobei das Antigen ein Tumorantigen oder Infektionsantigen oder ein Teil davon ist.

16. Effektive dendritische menschliche MUTZ-3 Zellen oder MUTZ-3 Zelllinie nach Anspruch 15, wobei das Tumorantigen oder Infektionsantigen ein Peptid, Protein, Lipid, Lipopeptid, Lipoprotein, Kohlenhydrat, Glykolipid, Glykopeptid, Glykoprotein, phosphoryliertes Protein, phosphoryliertes Peptid, posttranslational modifiziertes Protein oder ein Peptid ist oder das Tumorantigen von DNA oder RNA kodiert wird, mit der die effektiven dendritischen menschlichen MUTZ-3 Zellen transfiziert werden.

17. Arzneimittel, umfassend die effektiven dendritischen menschlichen MUTZ-3 Zellen oder die MUTZ-3 Zelllinie nach einem der Ansprüche 13, 14, 15 oder 16.

18. Effektive dendritische menschliche MUTZ-3 Zellen oder MUTZ-3 Zelllinie nach einem der Ansprüche 13, 14, 15 oder 16 zur Verwendung als Immuntherapeutikum.

19. Effektive dendritische menschliche MUTZ-3 Zellen oder MUTZ-3 Zelllinie nach einem der Ansprüche 13, 14, 15 oder 16 zur Verwendung bei der Prophylaxe oder Behandlung von Infektions-, Tumor- und/oder Autoimmunerkrankungen.

20. Effektive dendritische MUTZ-3 Zellen oder MUTZ-3 Zelllinie nach Anspruch 13 zur Verwendung zum Prozessieren und/oder Präsentieren von Antigenen.

21. Effektive dendritische MUTZ-3 Zellen oder MUTZ-3 Zelllinie nach Anspruch 20, wobei die Antigene Peptide, Proteine, Lipide, Lipopeptide, Lipoproteine, Kohlenhydrate. Glykolipide, Glykopeptide, Glykoproteine, phosphorylierte Proteine und/oder phosphorylierte Peptide sind.

22. Testsystem, umfassend die effektiven dendritischen MUTZ-3 Zellen oder die MUTZ-3 Zelllinie nach Anspruch 13.

23. Testsystem nach Anspruch 22, wobei das Testsystem zur Testung von immunaktivierenden, -inhibierenden und/oder -modulierenden Substanzen verwendet wird.

24. Testsystem nach Anspruch 22, wobei das Testsystem zur Testung von Tumorvakzinen oder den Einfluss von chemischen Stoffen, pharmakologischen Agenzien, Kosmetika oder Nahrungsmittel auf das Immunsystem verwendet wird.

## Claims

1. A method for the production of effective dendritic cells or cell lines, **characterized in that** cells from the CD124- and CD116-positive cell line MUTZ-3 are contacted simultaneously or in a sequentially deferred fashion with stimulatory molecules and the effective dendritic cells or cell lines are obtained, wherein GM-CSF, TNFα, LPS, PGE2, CD40 ligand, polyinosinic-polycytidylic acid (polylC), PMA, TGFβ1, IL-7, or IL-4 are used as stimulatory molecules.

2. The method according to claim 1, **characterized in that** cells or cell lines are produced as effective dendritic cells or cell lines which have a phenotype of interstitial dendritic cells, Langerhans dendritic cells, CD83-positive cells, immature dendritic cells and/or mature dendritic cells.

3. The method according to claim 1 or 2, **characterized in that** the CD124- and CD116-positive cell line or cells is/are CD34-positive.

4. The method according to any one of claims 1-3, **characterized in that** the gene for CD34 is introduced into the CD116- and CD124-positive cell line or cells.

5. The method according to any one of the preceding claims, **characterized in that** additional genes are introduced into the cell line or cells, which genes encode and/or express, for example, receptors for, or inhibitors of stimulatory molecules.

6. The method according to any one of the preceding claims, **characterized in that** at least one immunotherapeutic agent gene is introduced into the cell line or cells, wherein an immunotherapeutic agent gene is a gene which encodes a tumor antigen, a viral antigen, antigen of a parasite, bacteria or microorganisms.

7. The method according to any one of the preceding claims, **characterized in that** the cell line or cells are fused with other cells or cell lines.

8. The method according to any one of the preceding claims, **characterized in that** effective dendritic cell lines or cells having different activation and/or effector stages are obtained as a result of different stimulatory molecules, the dosage thereof and/or the order of contacting.

9. The method according to any one of the preceding claims, **characterized in that** the CD124- and CD116-positive cell line and/or effective dendritic cells or cell lines are caused to undergo apoptosis or necrosis, for example, by irradiating the cells.

10. The method according to any one of claims 1-9, **characterized in that** at least one suicide gene is incorporated in the CD124- and CD116-positive cell lines and/or effective dendritic cells or cell lines.

11. The method according to any one of the preceding claims, **characterized in that** effective dendritic cell lines or cells of the DC type 1 or DC type 2 are obtained as a result of different stimulatory molecules, the dosage thereof and/or the order of contacting.

12. The method according to any one of the preceding claims, **characterized in that** the effective dendritic cell lines or cells are contacted with said stimulatory molecules, thus obtaining effective mature dendritic cell lines or cells.

13. Effective dendritic human MUTZ-3 cells or MUTZ-3 cell line, obtainable by the method of any one of claims 1-12.

14. The effective dendritic human MUTZ-3 cells or MUTZ-3 cell line according to claim 13 loaded with at least one antigen.

15. The effective dendritic human MUTZ-3 cells or MUTZ-3 cell line according to claim 14, wherein the antigen is a tumor antigen or infection antigen or portion thereof.

16. The effective dendritic human MUTZ-3 cells or MUTZ-3 cell line according to claim 15, wherein the tumor antigen or infection antigen is a peptide, protein, lipid, lipopeptide, lipoprotein, carbohydrate, glycolipid, glycopeptide, glycoprotein, phosphorylated protein, phosphorylated peptide, post-translationally modified protein or peptide or the tumor antigen is encoded by DNA or RNA which is transfected into effective dendritic human MUTZ-3 cells.

17. Pharmaceutical composition comprising the effective dendritic human MUTZ-3 cells or the MUTZ-3 cell line according to claim 13, 14, 15 or 16.

18. The effective dendritic human MUTZ-3 cells or MUTZ-3 cell line according to claim 13 for use as immunotherapeutic agent.

19. The effective dendritic MUTZ-3 cells or MUTZ-3 cell line according to claim 13, 14, 15 or 16 for use in the prophylaxis or treatment of infectious, tumor and/or autoimmune diseases.

20. The effective dendritic human MUTZ-3 cells or MUTZ-3 cell line according to claim 13 for use in the processing and/or presentation of antigens.

21. The effective dendritic human MUTZ-3 cells or MUTZ-3 cell line according to claim 20, wherein the antigens are peptides, proteins, lipids, lipopeptides, lipoproteins, carbohydrates, glycolipids, glycopeptides, glycoproteins, phosphorylated proteins and/or phosphorylated peptides.

22. Test system comprising the effective dendritic MUTZ-3 cells or MUTZ-3 cell line according to claim 13.

23. The test system according to claim 22 for testing immuno-activating, immuno-inhibiting and/or immuno-modulating compounds.

24. The test system according to claim 22 for testing tumor vaccines or the influence of chemical compounds, pharmacological agents, cosmetics or foodstuff on the immune system.

## Revendications

1. Procédé de production de cellules ou de lignées de cellules dendritiques effectrices, **caractérisé en ce que** la lignée cellulaire CD124 et CD116 positive MUTZ-3 est mise en contact avec molécules stimulatrices, en même temps ou de manière séquentiellement décalée dans le temps, et **en ce que** les cellules ou lignées de cellules dendritiques effectrices sont obtenues en utilisant comme molécules stimulatrices du GM-CSF, du TNF-α, du LPS, de la PGE2, un ligand de CD40, de l'acide polyinosinique polycytidylique (poly-IC), du PMA, du TGF-β1, de l'IL-7, ou de l'IL-4.

2. Procédé selon la revendication 1, **caractérisé en ce que** les cellules ou les lignées de cellules dendritiques effectrices produites sont des cellules ou des lignées cellulaires qui possèdent un phénotype de cellules dendritiques interstitielles, de cellules dendritiques de Langerhans, de cellules CD83 positives, de cellules dendritiques immatures et/ou de cellules dendritiques matures.

3. Procédé selon l'une des revendications précédentes, **caractérisé en ce que** la lignée cellulaire ou les cellules CD124 et CD116 positives est/sont positive(s) pour le CD34.

4. Procédé selon l'une des revendications précédentes, **caractérisé en ce que** le gène du CD34 est introduit dans la lignée cellulaire ou dans les cellules CD 116 et CD124 positives.

5. Procédé selon l'une des revendications précédentes, **caractérisé en ce que** d'autres gènes par exemple codant pour et/ou exprimant des récepteurs ou des inhibiteurs de molécules stimulatrices sont introduits dans la lignée cellulaire ou les cellules.

6. Procédé selon l'une des revendications précédentes, **caractérisé en ce qu'**au moins un gène immunothérapeutique est introduit dans la lignée cellulaire ou les cellules, le gène immunothérapeutique étant un gène qui code pour un antigène tumoral, un antigène viral, un antigène de parasites, de bactéries ou de micro-organismes.

7. Procédé selon l'une des revendications précédentes, **caractérisé en ce que** la lignée cellulaire ou les cellules est fusionnée avec d'autres cellules ou lignées cellulaires.

8. Procédé selon l'une des revendications précédentes, **caractérisé en ce que** selon les différentes molécules stimulatrices, leur dosage et/ou la séquence de mise en contact, des lignées cellulaires ou cellules dendritiques effectrices de stades d'activation et/ou effecteurs différents sont obtenues.

9. Procédé selon l'une des revendications précédentes, **caractérisé en ce que** la lignée cellulaire CD124 et CD116 positive et/ou les cellules ou lignées de cellules dendritiques effectrices sont impliquées dans l'apoptose ou la nécrose, par exemple par irradiation des cellules.

10. Procédé selon l'une des revendications précédentes, **caractérisé en ce qu'**au moins un gène suicide est introduit dans la lignée cellulaire ou les cellules CD124 et CD116 positives et/ou dans les cellules ou lignées de cellules dendritiques effectrices.

11. Procédé selon l'une des revendications précédentes, **caractérisé en ce que** selon les différentes molécules stimulatrices, leur dosage et/ou la séquence de mise en contact, des lignées cellulaires ou cellules dendritiques effectrices DC type 1 ou DC type 2 sont obtenues.

12. Procédé selon l'une des revendications précédentes, **caractérisé en ce que** les lignées cellulaires ou cellules dendritiques effectrices sont mises en contact avec les molécules stimulatrices et **en ce que** des lignées cellulaires ou cellules dendritiques effectrices matures sont obtenues.

13. Cellules dendritiques effectrices humaines MUTZ-3 ou lignée cellulaire MUTZ-3 pouvant être obtenue(s) par un procédé selon les revendications 1 à 12.

14. Cellules dendritiques effectrices humaines MUTZ-3 ou lignée cellulaire MUTZ-3 selon la revendication 13, qui est/sont chargée(s) avec au moins un antigène.

15. Cellules dendritiques effectrices humaines MUTZ-3 ou lignée cellulaire MUTZ-3 selon la revendication 14, dans lesquelles l'antigène est un antigène tumoral ou un antigène infectieux, ou une partie d'un tel antigène.

16. Cellules dendritiques effectrices humaines MUTZ-3 ou lignée cellulaire MUTZ-3 selon la revendication 15, dans lesquelles l'antigène tumoral ou l'antigène infectieux est un peptide, une protéine, un lipide, un lipopeptide, une lipoprotéine, un glucide, un glycolipide, un glycopeptide, une glycoprotéine, une protéine phosphorylée, un peptide phosphorylé, une protéine ou un peptide modifié(e) après traduction, ou bien l'antigène tumoral est codé par de l'ADN ou de l'ARN avec lequel les cellules dendritiques effectrices humaines MUTZ-3 sont transfectées.

17. Médicament, comprenant les cellules dendritiques effectrices humaines MUTZ-3 ou la lignée cellulaire MUTZ-3 selon l'une des revendications 13, 14, 15 ou 16.

18. Cellules dendritiques effectrices humaines MUTZ-3 ou lignée cellulaire MUTZ-3 selon l'une des revendications 13, 14, 15 ou 16, destinées à être utilisées comme agent immunothérapeutique.

19. Cellules dendritiques effectrices humaines MUTZ-3 ou lignée cellulaire MUTZ-3 selon l'une des revendications 13, 14, 15 ou 16, destinées à être utilisées dans la prévention ou le traitement de maladies infectieuses, tumorales et/ou auto-immunes.

20. Cellules ou lignée de cellules dendritiques effectrices MUTZ-3 selon la revendication 13, destinées à être utilisées pour l'élaboration et/ou la présentation d'antigènes.

21. Cellules ou lignée de cellules dendritiques effectrices MUTZ-3 selon la revendication 20, dans lesquelles les antigènes sont des peptides, des protéines, des lipides, des lipopeptides, des lipoprotéines, des glucides, des glycolipides, des glycopeptides, des glycoprotéines, des protéines phosphorylées et/ou des peptides phosphorylés.

22. Système de test, comprenant les cellules ou la lignée de cellules dendritiques effectrices MUTZ-3 selon la revendication 13.

23. Système de test selon la revendication 22, dans lequel le système de test est utilisé pour le test de substances qui activent, inhibent et/ou modulent l'immunité.

24. Système de test selon la revendication 22, dans lequel le système de test est utilisé pour tester des vaccins antitumoraux ou l'effet de composés chimiques, d'agents pharmacologiques, de cosmétiques ou de produits alimentaires sur le système immunitaire.
